# EUROPEAN PATENT SPECIFICATION

(11) **EP 1 910 383 B1**
(45) Date of publication and mention of the grant of the patent: **16.09.2009**
(21) Application number: 06756301.5
(22) Date of filing: 26.05.2006
(51) Int. Cl.: C07D 513/04, C07D 487/04, A61K 31/55, A61P 35/02

(54) **BENZODIAZEPINE DERIVATIVES AND USES THEREOF IN MEDICAL FIELD**
BENZODIAZEPINDERIVATE UND ANWENDUNGEN DAVON AUF DEM MEDIZINISCHEN GEBIET
DÉRIVÉS DE BENZODIAZÉPINE ET UTILISATIONS DE CEUX-CI DANS LE DOMAINE MÉDICAL

(30) Priority: 03.08.2005 IT RM20050416
(43) Date of publication of application: 16.04.2008
(73) Proprietor: Universita' degli Studi di Roma " Tor Vergata", 00173 Roma (IT)
(72) Inventor: SILVESTRI, Romano, 00152 Roma (IT); MARFE', Gabriella, Via Orazio Raimondo 18, 00173 Rome (IT); ABRUZZESE, Elisabetta, Via Orazio Raimondo 18, 00173 Rome (IT); CATALANO, Gianfranco, Via Orazio Raimondo 18, 00173 Rome (IT); DI STEFANO, Carla, 00142 Roma (IT); NOVELLINO, Ettore, Via Orazio Raimondo 18, 00173 Rome (IT); SINIBALDI SALIMEI, Paola, Via Orazio Raimondo 18, 00173 Rome (IT); LA REGINA, Giuseppe, 00167 Roma (IT); LAVECCHIA, Antonio, Via Orazio Raimondo 18, 00173 Rome (IT)
(74) Representative: Gitto, Serena
(86) International application number: PCT/IT2006/000401
(87) International publication number: WO 2007/015280

(56) References cited:
- US-A- 6 156 746
- ARTICO, MARINO ET AL: "5H-Pyrrolo[1,2-b][1,2,5]benzothiadiazepin es (PBTDs): a novel class of non-nucleoside reverse transcriptase inhibitors" BIOORGANIC & MEDICINAL CHEMISTRY , 4(6), 837-850 CODEN: BMECEP; ISSN: 0968-0896, 1996, XP002396914
- SILVESTRI, ROMANO ET AL: "Synthesis and anti-HIV activity of 10,11-dihydropyrrolo[1,2- b][1,2,5]benzothiadiazepine-11-acetic acid 5,5-dioxide derivatives and related compounds" FARMACO , 51(6), 425-430 CODEN: FRMCE8, 1996, XP008068382
- SILVESTRI, ROMANO ET AL: "Heterocycles with a benzothiadiazepine moiety. 3. Synthesis of imidazo[5,1-d]pyrrolo[1,2-b][1,2,5]benzoth iadiazepine 9,9-dioxide" JOURNAL OF HETEROCYCLIC CHEMISTRY , 31(4), 1033-6 CODEN: JHTCAD; ISSN: 0022-152X, 1994, XP000576065
- STEFANCICH, GIORGIO ET AL: "Heterocycles with a benzothiadiazepine moiety. 2. Synthesis of 2-methyl-1,3,4,14b-tetrahydro-2H-pyrazino[ 2,1-d]pyrrolo[1,2- b][1,2,5]benzothiadiazepine 10,10-dioxide (tiaaptazepine)" JOURNAL OF HETEROCYCLIC CHEMISTRY , 31(4), 867-9 CODEN: JHTCAD; ISSN: 0022-152X, 1994, XP008068364
- STEFANCICH, GIORGIO ET AL: "Nonsteroidal antiinflammatory agents. VII. Synthesis and antiinflammatory activity of 5-methyl-10,11-dihydro-5H-pyrrolo[1,2-b][1 ,2,5]benzotriazepine- 11-acetic acid and its 10-aroyl derivatives" FARMACO , 45(7-8), 817-31 CODEN: FRMCE8; ISSN: 0014-827X, 1990, XP008068383
- STEFANCICH, GIORGIO ET AL: "Research on nitrogen containing heterocyclic compounds. XVI. Synthesis of 1,3,4,14b-tetrahydro-2,10-dimethyl-2H,10H- pyrazino[2,1-d]pyrrolo[1,2- b][1,2,5]benzotriazepine (1:1) maleate (10-methyl-10-azaaptazepine)" JOURNAL OF HETEROCYCLIC CHEMISTRY , 26(3), 745-6 CODEN: JHTCAD; ISSN: 0022-152X, 1989, XP002396917
- STEFANCICH, GIORGIO ET AL: "Research on nitrogen containing heterocyclic compounds; XIV. Derivatives of 5H-pyrrolo[1,2-b][1,2,5]benzotriazepine, a novel tricyclic ring system, by intramolecular cyclization of N-aryl-1H-pyrrol-1-amines" SYNTHESIS , (9), 757-9 CODEN: SYNTBF; ISSN: 0039-7881, 1983, XP002396918
- CHIMENTI, F. ET AL: "Compounds with antiblastic activity. LVII. Anthramycin and related compounds. VI. Synthesis of pyrrolo[1,2-b][1,2,5]benzothiadiazepine derivatives" FARMACO, EDIZIONE SCIENTIFICA , 29(8), 589-97 CODEN: FRPSAX; ISSN: 0430-0920, 1974, XP000576049

## Description

The present invention concerns benzodiazepine derived compounds, in particular pyrrolo [1,2-b] [1,2,5] benzothiadiazepines and pirrole [1,2-b][1,2,5] benzotriazepine, their uses in medical field, in particular like antitumoral agents.

Leukemia affects bone marrow cells, which are progenitors of leukocytes or white blood cells, characterized in that serious alterations of the mechanisms regulating the processes of cell synthesis and maturation. In fact immature cells reproduce rapidly and invasively by replacing in the marrow normal ones and tend with the time to invade other organs (spleen, liver, central nervous system).

Acute myelogenous leukemias (AML) originate from one or more genetic alterations involving the haemopoietic stem cell and are characterized by elevated proliferation and differentiation thereof and derived cell lines: myelogenous (granulocytic and monocytic), erythroid, megakaryocytic. Leuckemic cells, that is modified blastocytes, accumulate abnormally in the bone marrow resulting in a disorder or suppression of the normal hemapoietic cell differentiation and proliferation. Subsequently the modified blastocytes invade the peripheral blood infiltrating various organs. AML incidence is equal to approximately 3.5 cases for 100 000 inhabitants for year. AMLs can occur at any age, but their occurrence frequency increases with age; in fact AMLs constitute nearly the totality of the acute leukemias for the old humans. AMLs are classified as acute leukemias *de novo* when they occur like first disease, or secondary acute leukemias when they occur in patients previously exposed to chemicals, drugs, radiations or previous haemopathies. Moreover, AMLs are subdivided in groups based on morphologic (different staining for the cells of every group), cytochemical (evidence of enzymatic activities), immunophenotypic (evidence of surface and cytoplasmic specific marker using monoclonali antibodies) and cytogenetic (demonstration of specific chromosome translocations).

The more meaningful chromosome anomalies in AML are: translocation t (15; 17) (q22; q21); inversion inv (16) (p13; q22) and translocation t (16; 16) (p13; q22); translocation t (8; 21) (q22; q22); translocation t (9; 22) (q34; q11); anomalies involving chromosome 11q23; deletion of 5 or 5q chromosome, deletion of chromosome 7 or 7q and more complex rearrangements for the secondary acute leukemias.

Acute lymphoblastic leukemias (ALLs) represent a set of neoplastic clonal disorders originating from lymphoid progenitors in the marrow, thymus and the lymph nodes. Approximately 80% of ALLs constitute malignant proliferations of cells B precursors, while 20% comprise cases deriving from involvement of cell T precursors. The incidence of ALLs is highest in the children and the young people under 15 years. Cytogenetic analysis of ALLs revealed the presence of clonal chromosome aberrations in 90% of the patients. Among these, translocations t (9; 22) (q34; q11) and t (the 8; 14) (q24; q32) are particularly important because they are involved in other leukemia forms and are emblematic of the two ways through which the activation of a proto-oncogene can occur.

Chronic myelogenous leukemia (CML), is the most studied myeloproliferative disorder resulting from the neoplastic transformation of the pluripotent stem cell and characterized by prevailing hyperplasia of the granulocyte line.

CML was first human disease wherein a specific anomaly of the karyotype at level of Philadelphia (Ph) chromosome. Therefore, the chronic myelogenous leukemia is the result of an acquired stem cell DNA damage. The DNA alteration confers to these stem cells a growing advantage in comparison to normal cells; the consequence is an uncontrolled growth of leukocytes in the blood (Sawyers CL., 1999).

The Philadelphia (Ph) chromosome is the characteristic marker of the Chronic Myelogenous Leukemia (LMC). It results from the reciprocal translocation between 9 and 22 chromosomes. This translocation, that is (the 9; 22), determines at molecular level the formation of a fusion gene between the bcr (located on chromosome 22) and abl (on chromosome 9) genes. Bcr-abl hybrid gene encodes for a protein of molecular weight 210 Kd (p210) from which mechanism of leukemic transformation in the LMC originates. p210 is detectable in nearly all the LMC cases and in approximately 50% of the cases of Ph positive lymphoblastic acute leukemia (LAL). There are also other forms of bcr-abl gene fusion characterized by a different extension of bcr component and encoding for fusion proteins of different molecular weight: namely, p190, which is observed in 50% of LAL cases, and p230, which is associated to a LMC clinical picture of indolent course LMC (Lugo TG, et al., 1990).

The abl gene on 9 chromosome belongs to the class of not receptor tyrosine kinases (TK), that is enzymes phosphorylating substrates at level of tyrosine residues, but not possessing receptor functions at level of cellular membrane. It is thought that abl is able to activate the transduction of mitogenic signal by activating Ras and at cascade other kinases, which in turn activate transcription factors triggering mechanisms of cellular proliferation. In t (the 9; 22) abl is nearly entirely translocated on the chromosome 22 and the hybrid protein resulting from bcr-abl hybrid gene maintains high and constituently activated TK activity. From above the leukemia generation potential of the hybrid protein results. Such leukemia potential is expressed through: 1) interferences with the proliferative activity of the haemapoietic cells; 2) alteration of the adhesion mechanisms and responsiveness to the proliferation regulating factors; 3) reduced apoptosis (Daley GQ, et al., 1990).

Since TK activity of the bcr-abl protein is an essential moment in the pathogeneses of LMC, various molecules able to inhibit abl kinase activity have been designed. Among the products with abl inhibiting specific activity it has been discovered that the most effective for the Ph positive leukemias is the compound known as STI571 ((STI: Signal Transduction Inhibitor), or mesylated Imatinib, or, in Italy, known with trade name Glivec (Cortes J, et al., 2004).

It is a relatively simple molecule, derived from the 2-phenyl amino pyrimidine, suitable to occupy competitively the ATP occupied pocket in the bcr-abl protein, thus inhibiting substrates phosphorylation. Recent studies about this drug outlined the problems of drug-resistance: Hochhaus A, et al., 2004; Weisberg E & Griffin JD., 2000; Mahon F.X., et al., 2000; Bhatia R, et al., 2003; Goldman JM & Melo JV., 2003; Ottmann et al., 2002; Lydon NB & Druker BJ., 2004; Buchdunger E, et al., 2000; Okuda K, et al., 2001; Sawyers CL, et al., 2002; Talpaz M, et al., 2002; O'Brien SG, et al., 2003; Kantarjian H, et al., 2002; Druker, BJ, 2002; 2001; Kantarjian H et al., 2002; O'Brien SG, et al., 2003; Wu CJ, et al., 2002; Azam, M, et al., 2003) and the possible underlying mechanisms:
a) in some myelogenous leukemia forms it can occur that the BCR-ABL anomalous gene, which triggers neoplasia, becomes "amplified" in parallel with the development of the disease;
b) other times a protein associated to the drug activity (AGP) can chelate STI571 molecules before these to be bound to neoplastic cells;
c) some studies found that BCR-ABL gene itself is changed so as to not to bind the drug and not to be sensitive to activity thereof any longer.

In fact, one of the major problems of the antitumoral chemotherapy concerns the drug resistance. The acquired resistance in the chronic myelogenous leukemia seems to be the result of graduated suppression of the apoptosis. New finds in the field of the apoptosis confirms that various antitumoral drugs would act by apoptosis mechanism and the resistance would be acquired through by-passing the apoptosis process as consequence of mutations in the tumour cells.

An ideal apoptosis drug would act selectively on the onocogene cells, i.e. inducing apoptosis only in the tumour cells without increase of the apoptosis in normal ones; further it would avoid the drug resistance.

Among the substances known for the apoptotic activity, there are several antitumoral drugs, as camptothecin, etoposide, anthracycline antibiotics like daunoribucin and doxorubicin (adriamycin), taxol, vincristine, 5-fluorouracyl and cisplatinum. In laboratory experiments camptothecin, etoposide, daunoribucin and doxorubicin proved to be able to induce apoptosis in cancer cells from the haemopoietic system, like HL-60 and Jurkat cells. Therefore, any new substance suitable to induce apoptosis in similar cell lines has the potential to be used in antitumoral therapy.

Further studies found that BCR-ABL gene itself is mutated so as to not bind the drug and not to be sensitive to its activity any longer. Today two new drugs, BMS-354825 and AMN107, are being studied: the first one currently is being tested on patients suffering from chronic myelogenous leukemia, the second on rats suffering from chronic myelogenous leukemia and it seems that they do not show activity (*in vivo* on rats suffering from chronic myelogenous leukemia) on a specific mutation of BCR-ABL protein (O'Hare T., ET becomes ill you of chronic mieloide leukemia) on one to, 2005; Burgess MR., et al., 2005).

Thyroid malignant neoplasias result from the epithelial structures constituting the glandular parenchyma or from the cells of therein distributed parafollicular system. In fact, thyroid carcinomas are classifiable in four main hystotypes: follicular, papillary, anaplastic and medullary. The more differentiated forms (papillary and follicular) originate from the thyroid epithelial cells, anaplastic from presumably from one of the differentiated hystotypes, while the medullary one originates from calcitonin secreting parafollicular cells (cells C).

Differentiated carcinomas, i.e. papillary and follicular, are relatively rare neoplasias, because they constitute approximately 1-1,5% of all the human carcinomas; anaplastic carcinoma, highly malignant tumour, occurs more frequently after 60 year age and the feminine sex is preferred; finally medullary carcinoma, that represents 5-10% of all thyroid neoplasias, occurs in familiar or sporadic mode, often associated to endocrine multiple neoplasias. Pathogenesis of the familiar form is associated to mutations in the germ line of RET proto-oncogene, localized in the proximal region of the long branch of chromosome 10, encoding for a transmembrane receptor with tyrosine-kinase activity.

Molecular events involved in thyroid carcinogenesis include activation of RET/PTC, that is chimeric genes, generated by the fusion of the tyrosine-kinase domain of RET gene with 5' end region of other genes. Various RET/PTC isoforms have been identified and they differ from each other for the fusion type: namely the fusions occur with H4 (or D10S170) and RFG genes in RET/PTC1 and RET/PTC3, respectively..

Recently 3 tyrosine-kinase inhibitors, i.e. PP1 and PP2 and 4-anilinequinazoline ZD6474 have been identified, which have a strong inhibiting activity with respect to RET kinase and are able to block the transforming activity of RET/C634R and RET/M918T mutants.

The activity of such inhibitors has been tested against remaining mutant of the RET intracellular domain. In vivo self-phosphorylation of RET/E768D, RET/L790F, RET/Y791F, RET/A883F and RET/S891A mutants is inhibited effectively by PP1, PP2 and ZD6474, while RET/V804L and RET/V804M mutants are less sensitive to the action of inhibitors. The IC₅₀ value of ZD6474 for kinase activity of the mutants of the residue V804 *in vitro* increases by fifty times. 1 µl of ZD6474 reduces strongly the proliferation of RET/C634R transformed RAT1 fibroblasts, while at the same concentration there is no effect on the growth of the RET/V804L and RET/V804M transformed cells. The substitution of V804 residue with a smaller amino acid like glycine makes kinase more *in vivo* sensitive to PP1 and ZD6474; IC₅₀ of ZD6474 *in vitro* for V804G mutant is diminished by five times (20 nM).

From above it can be deduced that the V804 residue of RET is important not only for the kinase sensitivity to the inhibitors, but the natural mutants of V804 residue (V804L and V804M) are partially resistant to inhibition by PP1, PP2 and ZD6474 (Grieco M, et al., 1990; Pierotti BUT, et al., 1996; Santoro M, et al., 1994;)

The search for new agents suitable to activate or modulate some phases of the apoptosis process can allow an important progress in the therapy of the tumour diseases associated to progressive chemotherapy resistance, with inevitably fatal final outcome, to be carried out.

In the light of above it is apparent the need to provide new active principles to be used advantageously as antitumoral agents, in order to overcome the problem of the drug resistance associated to the currently used chemotherapeutics.

The authors of the present invention found now a new class of synthetic derivative compounds of benzothiadiazepines to be used advantageously as antitumoral and pro-apoptotic agents and, in particular, for the treatment of leukemia, which have been proved able to by-pass the agent suppressor of the apoptosis Bcr-Abl or carcinomas.

In the earlier stage of the study, the authors carried out *in vitro* studies on three different K562 cell lines. The authors of the present invention found now a new class of synthetic derivative compounds of benzothiadiazepines to be used advantageously as antitumoral and pro-apoptotic agents, in particular for the treatment of leukemia, which proved able to by-pass the agent suppressor of the apoptosis Bcr-Abl or carcinomas.

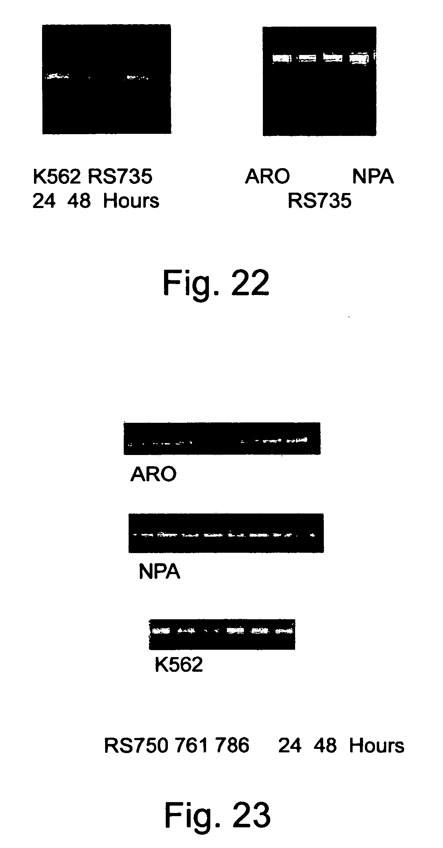

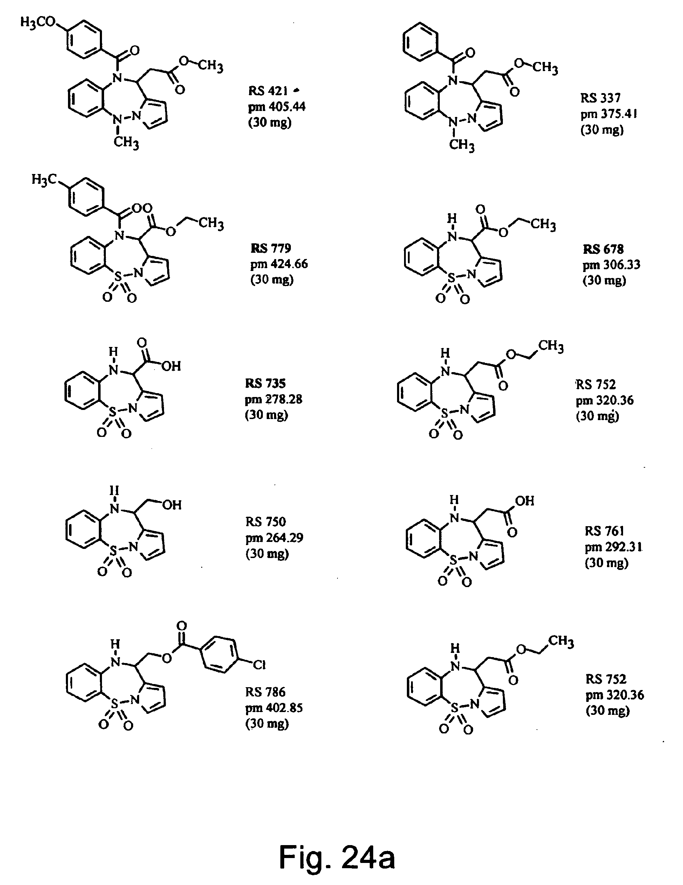

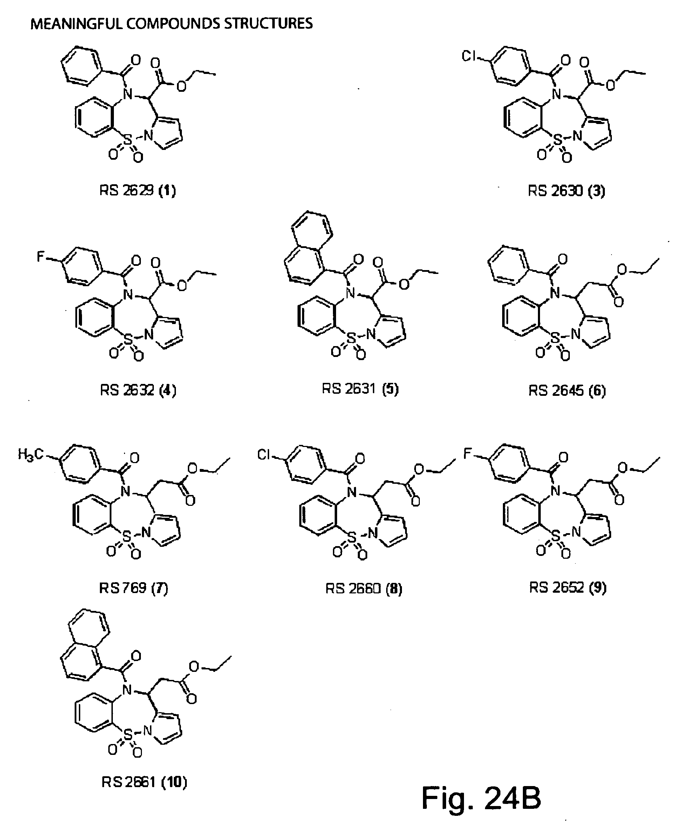

In an earlier step of the study, the authors carried out *in vitro* studies on five different hematological K562 cell lines (human erythroleucemic cell line), HL60 (human acute promyelocytic leukemia cell line), Jurkat (cell line from acute lymphoblastic T variant leukemias) U937 (acute promyelocitic leukemia cell line) and Gilvec resistant K562 (Morotti A., et al., 2006) treated using synthetic compounds as reported in Figures 22A and 22B, in order to induce apoptosis and explain the activity mechanism using molecular biology techniques (Western blotting, tyrosine kinase assays).

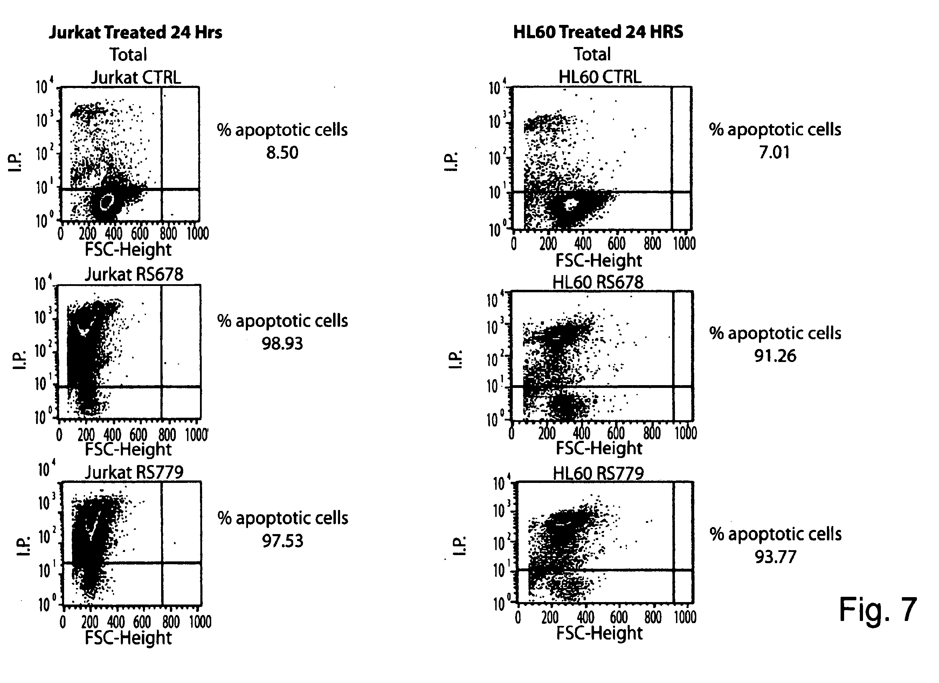

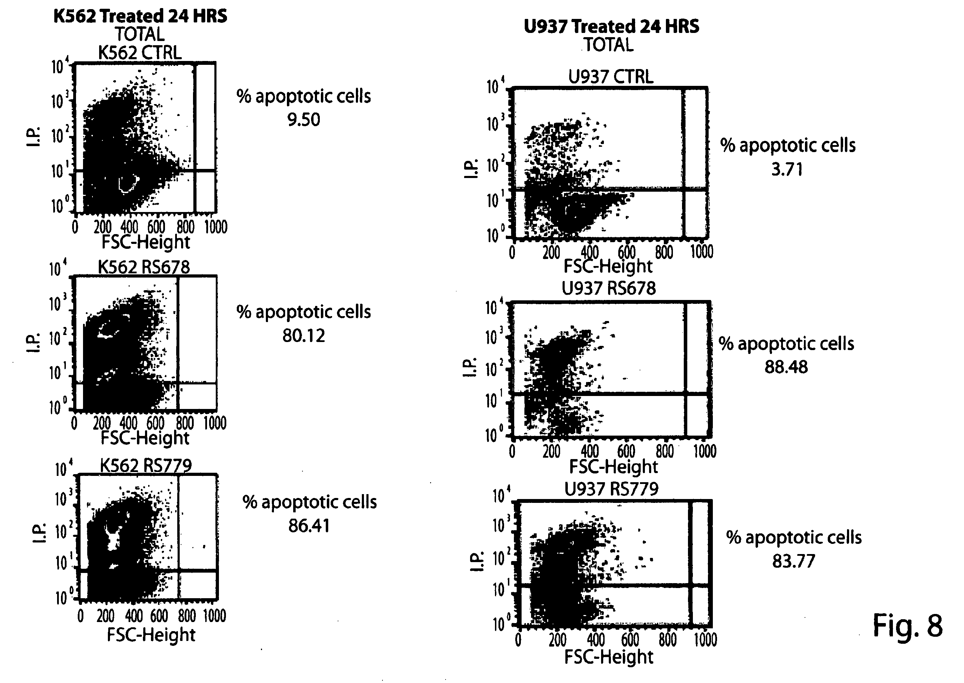

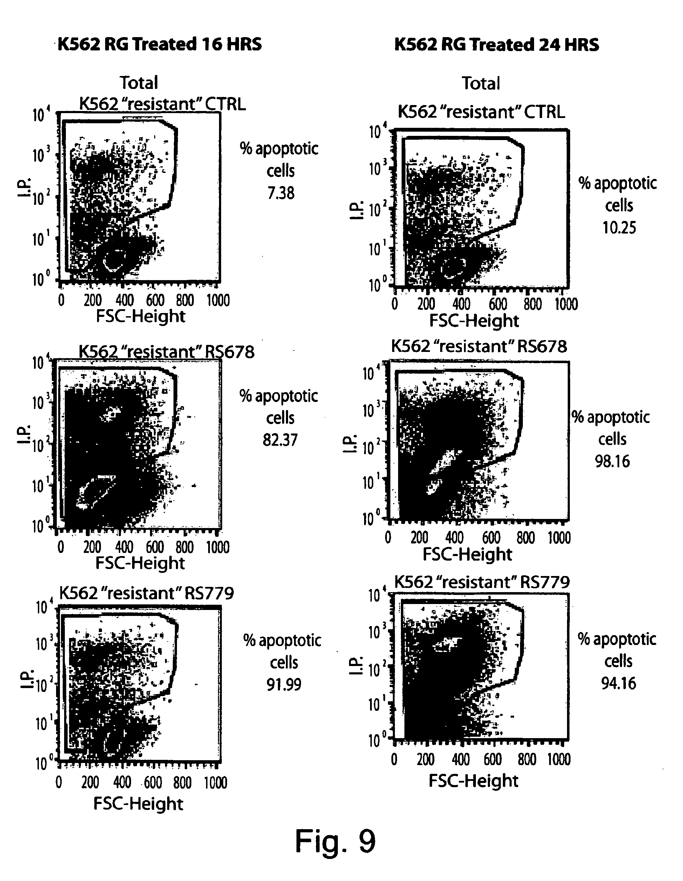

RS678 and RS 779 compounds are able to induce the apoptosis after 8 hours in all the tested hematological lines, with exception of Glivec resistant K562 in which case 16 hours are necessary for the induction of the apoptosis (Figures 7, 8 and 9).

In a second experimental phase the authors tested *in vitro* two compounds according to the invention (RS-678 and RS-779) against two mixed lymphocytic cultures of peripheral and medullary blood from patients suffering from chronic myelogenous leukemia at onset, some of which patients treated with Glivec, to which they became resistant, successively the same were treated according to the new experimental protocol with anti-tyrosine-kinase BMS-354825 in order to obtain the attenuation of the clinical symptomatology.

Subsequently the compounds were successfully tested *in vitro* against OVCAR3 ovary and ARO and NPA thyroid cell lines.

Benzodiazepine drugs are well known for their hypnotic-sedative, anxiolytic and muscle-relaxant activity. It is known that benzodiazepines affect the cell growth and differentiation (Kamal A., et al., 2004; Bednarski JJ, et al., 2004; Sutter AP, et al., 2002; Blatt NB, et al., 2002).

It is known from patent literature the use of benzothiadiazepine derivatives, for various uses in medical field like antiepileptics (imidazo [2,1-b] [1,3,5] benzothiadiazepines, US 4444688; US 4062852), antibacterials (pyrimido [1,2-c] [1,3,5] benzothiadiazepines, GB 1476684), diuretics and hypotensives (pyrrolo (1,2-b) [1,2,5] benzothiadiazepine 5,5 dioxide, US 3506646), hypolipidemics (WO 03091232), anti-depressants (US 3453266); osteoporosis (JP 2138272); antitumoral agents (N-2-imidazolyl alkyl substituted 1,2,5-benzothiadiazepine-1,1-dioxide, US 6156746) and benzo-pyrido or dipyrido thiadiazepine (WO 2004/069843).

US patent No. 6156746 discloses inhibitors of S-farnesyl protein transferase or prenyl transferase and the activation of Ras protein by means of the aforesaid post-transduction modifications, which can be used like antitumoral compounds, because they are suitable to induce apoptosis. N-2-imidazolyl alkyl substituted 1,2,5-benzothiadiazepine-1,1-dioxide compounds described in the document are useful for the treatment of several cancer types wherein Ras is involved, including carcinoma (among which there are colon, lungs, thyroid, pancreas, ovary carcinomas) and the tumours of the lymphoyd line, as for example acute lymphocytic leukemia, T cell lymphoma, B cell lymphoma, and chronic myelogenic leukemia. More particularly in the formula of above reported benzothiadiazepine compounds the five C atom ring is imidazolyl ring bound to N of seven member ring containing SO₂ group through a side chain.

US patent No. 3506646 discloses the process for the preparation of pyrrolo [1,2-b] [1,2,5] benzothiadiazepine 5,5, dioxide and uses thereof as diuretic and hypotensive agents. The general formula containing pyrrolo ring fused to seven member ring containing SO₀ group is without substituents in the seven member heterocycle ring.

It is therefore an object of the present invention the use of derivative compounds of benzodiazepines, particularly pyrrolo [1,2-b] [1,2,5] benzothiadiazepine and 5H-pyrrolo [1,2-b] 1,2,5 ][ benzotriazepine, of formula (i): 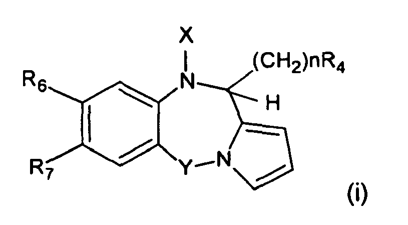 wherein
Y it is SO₂ or NR, wherein R is H or C₁-C₆ alkyl;
X is H, C₁-C₁₂ alkyl, -CO, -SO, -SO₂, or - CO-R₂, SO-R₂, SO₂-R₂, wherein R₂ is selected from H, C₁-C₁₂ alkyl, (C₃-C₈ cycloalkyl) C₀-C₆ alkyl, C₂-C₆ alkenyl, C₂-C₆ alkynyl, C₂-C₆ alkoxycarbonyl, phenyl, benzyl, naphtyl, biphenyl, or heterocycle, each para-, meta- or ortho-substituted independently of each other with 0 to 3 substituents selected from halogen, -CN, -NH₂, -OH, -NO₂, COOR₃, wherein R₃ is selected from H, C₁-C₁₂ alkyl, (C₃-C₈ cycloalkyl) C₀-C₆ alkyl, C₁-C₆ alkoxy, C₂-C₆ alkenyl, C₂-C₆ alkynyl, or C₂-C₆ alkoxy carbonyl; phenyl, benzyl, naphtyl, biphenyl, or heterocycle,
n is 0-6;
R₄ is selected from H, OH, COOH, CN, C₁-C₆ alkyl, C₁-C₆ alkoxy, C₂-C₆ alkenyl, C₂-C₆ alkynyl, C₂-C₆ alkoxycarbonyl, -CO, -SO, - SO₂, or -CO-R₅, SO-R₅, SO₂-R₂. -OCO-R₅, OSO-R₅, OSO₂-R₅, COOR₅, SOOR₅, SO₂OR₅, NHR₅, NHCOR₅, NHSO₂R₅, SR₅, SCOR₅, wherein R₅ is selected from H, C₁-C₆ alkyl, C₁-C₆ alkoxy, C₂-C₆ alkenyl, C₂-C₆ alkynyl, or C₂-C₆ alkoxycarbonyl, phenyl, benzyl, naphtyl, biphenyl, or heterocycle, each para-, meta- or ortho-substituted independently of each other with 0 to 3 substituents selected from halogen, -CN, -NH₂, -OH, -NO₂, COOR_{5'}, wherein R₅ is selected from H, C₁-C₁₂ alkyl, (C₃-C₈ cycloalkyl) C₀-C₆ alkyl, phenyl, benzyl, naphtyl, biphenyl, or heterocycle, C₁-C₆ alkyl, C₁-C₆ alkoxy, C₂-C₆ alkenyl, C₂-C₆ alkynyl, or C₂-C₆ alkoxy carbonyl;
R₆ and R₇ independently of each other are selected from the group consisting of H, OH, halogen, CN, NH₂, NO₂, COOR₃, C₁-C₆ alkyl, C₁-C₆ alkoxy, C₂-C₆ alkenyl, C₂-C₆ alkynyl, and C₂-C₆ alkoxy carbonyl;
for the preparation of a medicament for the treatment of solid or liquid tumours.

In the context of the present invention heterocycle means pyrrolo, furan, thiazole, pyridine, indole, benzofuran.

In a preferred embodiment for the use of the compounds according to the invention, n is 0 or 1 and R₄ is COOR₅, wherein R₅ is defined as above, preferably R₃ is C₁-C₆ alkyl, more preferably methyl or ethyl.

In another embodiment for the use of the compounds according to the invention, n is 0 or 1 and R₄ is COOH.

In a preferred embodiment for the use of the compounds according to the invention, n is 1 and R₄ is OH.

In a further embodiment for the use of the compounds according to the invention, n is 1 and R₄ is 4-chlorophenyl carbonyloxy.

The compounds according to the present invention are preferably compounds wherein X is H.

Alternatively in a preferred embodiment the compounds according to the present invention, X is -CO-R₂, wherein R₂ has the meaning as previously expressed, and preferably is phenyl or naphtyl. More preferably R₂ is phenyl monosubstituted in para position with CI, F, OCH₃, or CH₃.

Preferably, in all the aforesaid embodiments for the compounds according to the invention, R₆ and R₇ in the formula (i) are H.

In a still preferred embodiment for the compounds according to the invention, Y in the general formula (i) is SO₂. Preferably, said pyrrolo [1,2-b][1,2,5]benzothiadiazepine compounds are selected from the group consisting of:
ethyl 10,11-dihydropyrrolo [1,2-b] [1,2,5] benzothiadiazepine-11-carboxylate 5,5-dioxide (RS 678);
ethyl 10-(4-tolyl)-10,11-dihydropyrrolo [1,2-b][1,2,5] benzo thiadiazepine-11-acetate 5,5-dioxide (RS-779);
10,11-dihydropyrrolo [1,2-b] [1,2,5] benzothiadiazepine-11-carboxylic 5,5-dioxide acid (RS-735);
10,11-dihydropyrrolo [1,2-b] [1,2,5] benzothiadiazepine-11-acetic 5,5-dioxide acid (RS-791);
10,11-dihydropyrrolo [1,2-b] [1,2,5] benzothiadiazepine-11-methanol 5,5-dioxide (RS-750);
ethyl 10,11-dihydro-10-(4-tolyl) pyrrolo [1,2-b] [1,2,5]- benzothiadiazepine acetate 5,5-dioxide (RS-769);
ethyl 10,11-dihydro-10-(benzoyl) pyrrolo [1,2-b] [1,2,5] benzothiadiazepine-11-carboxylate 5,5-dioxide (RS-2629);
ethyl 10,11-dihydropyrrolo [1,2-b] [1,2,5] benzothiadiazepine-11-acetate 5,5-dioxide (RS-752);
ethyl 10,11-dihydro-10- (benzoyl) pyrrolo [1,2-b] [1,2,5] benzothiadiazepine-11-acetate 5,5-dioxide (RS-2645);
10,11-dihydropyrrolo [1,2-b] [1,2,5] benzothiadiazepine-11-acetic 5,5-dioxide acid (RS-761);
11-(4-Chloro phenybenzoyloxy methylene) 10,11-dihydro pyrrolo [1,2-b] [1,2,5] benzothiadiazepine 5,5-dioxide (RS-786);
ethyl 10,11-dihydro-10- (4-chlorobenzoyl) pyrrolo [1,2-b] [1,2,5] benzothiadiazepine-11-carboxylate 5,5-dioxide (RS-2630);
ethyl 10,11-dihydro-10-(1-naphtoyl) pyrrolo [1,2-b][1,2,5] benzothiadiazepine-11-carboxylate 5,5-dioxide (RS-2631);
ethyl 10,11-dihydro-10- (4-fluorobenzoyl) pyrrolo [1,2-b] [1,2,5] benzothiadiazepine-11-carboxylate 5,5-dioxide (RS-2632);
ethyl 10,11-dihydro-10-(4-chlorobenzoyl) pyrrolo [1,2-b] [1,2,5] benzothiadiazepine-11-acetate 5,5-dioxide (RS-2660);
ethyl 10,11-dihydro-10-(1-naphtoyl) pyrrolo [1,2-b] [1,2,5] benzothiadiazepine-11-acetate 5,5-dioxide (RS-2661);
   or
ethyl 10,11-dihydro-10- (4-fluorobenzoyl) pyrrolo [1,2-b] [1,2,5] benzothiadiazepine-11-acetate 5,5-dioxide (RS-2652).

According to an alternative preferred embodiment in the general formula Y is NR, and R is CH₃. Preferably, said pyrrolo [1,2-b] [1,2,5] benzotriazepine compounds are selected from the group consisting of
methyl 10,11-dihydro-5-methyl-5H-pyrrolo [1,2-b] [1,2,5]-benzotriazepine-11-acetate;
methyl 10,11-dihydro-5-methyl-10-benzoyl-5H-pyrrolo [1,2-b] [1,2,5]-benzotriazepine-11-acetate (RS-337)
or methyl 10,11-dihydro-5-methyl-10-(4-metoxybenzoyl)-5H-pyrrolo [1,2-b][1,2,5]-benzotriazepine-11-acetate (RS-421).

Preferably, said solid tumours are carcinomas, selected from the group consisting of ovary, thyroid, colon, pancreas, breast, gastric, prostate, pulmonary carcinomas. Said liquid tumours are selected from leukemias. Preferably, the leukemia is chronic myelogenous leukemia.

According to an alternative embodiment the leukemia is acute myelogenous leukemia (AML). Alternatively said leukemia is acute lymphoblastic leukemia (ALL).

The present invention has as further object the use of a pharmaceutical composition comprising at least one of above disclosed compounds, as active principles, together with one or more pharmacologically acceptable adjuvants and/or excipients for the treatment of the solid or liqui tumours. Preferably, said solid tumours are carcinomas, selected from the group consisting of ovary, thyroid, colon, pancreas, breast, gastric, prostate, pulmonary carcinomas. Said liquid tumours are selected from leukemias. Preferably, the leukemia is chronic myelogenous leukemia (AML). Alternatively said leukemia is acute lymphoblastic leukemia (ALL).

The present invention concerns also the derivatives compounds of benzodiazepines of formula (i), said compounds being selected from the group consisting of:
ethyl 10,11-dihydro-10-(benzoyl) pyrrolo [1,2-b] [1,2,5] benzothiadiazepine-11-carboxylate 5,5-dioxide (RS-2629);
ethyl 10,11-dihydro-10- (benzoyl) pyrrolo [1,2-b] [1,2,5] benzothiadiazepine-11-acetate 5,5-dioxide (RS-2645);
ethyl 10,11-dihydro-10- (4-chlorobenzoyl) pyrrolo [1,2-b] [1,2,5] benzothiadiazepine-11-carboxylate 5,5-dioxide (RS-2630);
ethyl 10,11-dihydro-10-(1-naphtoyl) pyrrolo [1,2-b][1,2,5] benzothiadiazepine-11-carboxylate 5,5-dioxide (RS-2631);
ethyl 10,11-dihydro-10- (4-fluorobenzoyl) pyrrolo [1,2-b] [1,2,5] benzothiadiazepine-11-carboxylate 5,5-dioxide (RS-2632);
ethyl 10,11-dihydro-10-(4-chlorobenzoyl) pyrrolo [1,2-b] [1,2,5] benzothiadiazepine-11-acetate 5,5-dioxide (RS-2660);
ethyl 10,11-dihydro-10-(1-naphtoyl) pyrrolo [1,2-b] [1,2,5] benzothiadiazepine-11-acetate 5,5-dioxide (RS-2661);
   or
ethyl 10,11-dihydro-10- (4-fluorobenzoyl) pyrrolo [1,2-b] [1,2,5] benzothiadiazepine-11-acetate 5,5-dioxide (RS-2652).

The present invention now will be described by way of illustration, not limitation, according to preferred embodiments thereof with particular reference to the figures of the enclose drawings, wherein:
figure 1 shows the DNA fragmentation in K562 leukemic cell line (a and B panels) by means of RS-678, RS-779 compounds; RS-735 compound is not able to produce the DNA fragmentation in such cell line; the fragmentation does not occur in R2C cell line (rat Leydig tumour) (panel C) (a benzodiazepine receptor rich cell line) treated with RS-678 and RS-779 at high concentrations;
figure 2, panels A and B, shows the effect of 5 µM, 10 µM, 15 µM concentrations of compounds RS-678 and RS-779, respectively, on the inhibition of the cell proliferation in comparison to the control;
figure 3 shows Western blot which detects the activation of caspases 8 and 9 following the treatment of the leukaeimic K562 cell line with RS-678 and RS-779 compounds;
figure 4 shows Western blot which detects the down-regulation of the bcr-abl protein after 16 hours of treatment with RS-678 and RS-779 compounds, and tyrosine-kinase assay, wherein changes in the phosphorylation pattern of the aforesaid protein are not observed;
figure 5 shows apoptosis morphology, cytoplasmic vacuolization, chromatin condensation;
figure 6 shows the formation of the membrane enveloped nuclear bodies and cytoplasmic vacuolization;
figures 7-9 show the results of cytofluorimetric analysis (FSC) to evaluate the apoptosis percentage after 16 and 24 hours of K562, Glivec resistant K562, U937, Jurkat and HL60 treated with pro-apoptotic RS678 and RS779 agents.
figure 10 shows the fragmentation of the DNA extracted from mixed lymphocytic cultures of 10 CML affected patients treated with 10 µM RS-678;
figure 11 shows the fragmentation of the DNA extracted from mixed lymphocytic cultures of 10 CML affected patients treated with 10 µM RS-779;
figures 12-14 show the DNA fragmentation on samples from patients treated with 10 µM RS-678, RS-779, RS 752 after 24, 48 and 72 hours; some of these chronic myelogenous leukemia affected patients treated with GLIVEC, which they then became resistant to, subsequently have been subjected to new experimental protocol with anti-tyrosine-kinase BMS-354825.
figure 15 shows Western blot of a sample treated with RS-779 concerning the expression and activity thereof after treatment for 24 and 48 hours;
figure 16 shows DNA fragmentation of various cell lines, i.e. OVCAR3 ovary, NPA papillary thyroid and ARO anaplastic carcinomas thyroid treated with RS-678 and RS-779 at 10 µM concentration for 24 and 48 hours;
figure 17 shows DNA fragmentation of ARO anaplastic thyroid cell line carcinoma treated with different synthetic compounds, namely RS-337, RS-421, RS-752, RS-2645, RS-2652, RS-2660, RS-2661, RS-2629, RS-2630, RS-2631, RS-2632, for 24 and 48 hours;
figure 18 shows DNA fragmentation of K562 leukemic cell line carcinoma treated with different synthetic compounds, namely RS-337, RS-421, RS-752, RS-2645, RS-2652, RS-2660, RS-2661, RS-2629, RS-2630, RS-2631, RS-2632 at 10 µM concentration for 24 and 48 hours;
figure 19 shows DNA fragmentation of NPA papillary thyroid cell line carcinoma treated with different synthetic compounds, namely RS-337, RS-421, RS-752, RS-2645, RS-2652, RS-2660, RS-2661, RS-2629, RS-2630, RS-2631, RS-2632 at 10 µM concentration for 24 and 48 hours;
figure 20 shows that the fragmentation of the DNA is not present in cellular lines K562, ARO and NPA treated with RS-735 for 24 and 48 hours;
figure 21 shows that the DNA fragmentation is not present in K562, ARO and NPA cell lines treated with other compounds RS-750, RS-761, RS-786, for 24 and 48 hours;
the figures 22A and 22B show benzodiazepine pyrrolo [1,2-b] [1,2,5] benzothiadiazepine (RS-735, RS-750, RS-752, RS-761, RS-786, RS-779, RS-678, RS-769, RS-2629, RS-2630, RS-2631, RS-2632, RS-2645, RS-2652, RS-2660, RS-2661), and pyrrolo [1,2-b][1,2,5] benzotriazepine (RS-421, RS- 20, RS-337) derivatives preferred as apoptosis inducers.

### EXAMPLE 1: Chemical processes for the preparation of benzodiazepine pyrrolo [1,2-b] [1,2,5] benzothiadiazepine and pyrrolo [1,2-b] 1,2,5][benzotriazepine derivatives

1- [(2-amminophenyl) sulfonil] - 1 H-pyrrolo (15) is prepared by reaction of 2-aminobenzenesulfonamide (13) with 2,5-dimetoxy tetrahydrofuran in boiling glacial acetic acid according to Clauson-Kaas reaction for the synthesis of pyrrolo (Clauson-Caas, et al., 1952; Elming et al, 1952) and subsequent reduction of nitro group with powdered iron in acetic acid according to the method reported by Chimenti et al, 1974.

In particular, to a solution of 2-nitrobenzenesulfonamide (1 g, 4,9 mmol) in glacial acetic acid (4 ml) 2,5-dimetoxytetrahydrofuran (0,65 g, 4,9 mmol, 0,64 ml) was added and the mixture was refluxed midnight. Acetic acid was evaporated under reduced pressure, then water (20 ml) and 20 % sodium hydroxide solution were added. After extraction with chloroform the organic layer was shaken with sodium chloride saturated solution, dried over anhydrous sodium sulfate and evaporated to dryness. The crude product was purified by crystallization from toluene/ligroin to yield 1-[(2-Aminophenyl) sulfonil]-1H-pyrrolo (15) (1,8 g, 73 % yield), melting point 52-54°C after re-crystallization from toluene/ligroin.

Compound (15) was subjected to Pictet-Spengler reaction (Pictet, et al, 1911) with ethyl 2,2-dietoxyacetate in anhydrous ethanol in the presence of 4-toluensulfonic acid (PTSA) as catalyst to give ethyl benzothiadiazepine-11-carboxylate 10,11-dihydropyrrolo [1,2-b][1,2,5] benzothyadiazepine-11-carboxylate 5,5-dioxide (RS 678) (16). Particularly, compound (16) was obtained as below: a mixture of 1-[(2-Aminophenyl) sulfonil]-1H-pyrrolo (15) (9,4 g, 0,042 mol), ethyl dimetoxyacetal glioxylate (11,10 g, 0,063 mol), 4-toluensulfonic acid monohydrate (PTSA) (8 g, 0,042 mol) was reflux heated in anhydrous ethanol (20 ml) midnight. PTSA (3,7 g, 0.021 mol) was added by heating for further 4 hours. After cooling the mixture was poured over ice, extracted with ethyl acetate, washed with sodium chloride saturated solution, dried over anhydrous sodium sulfate and the solvent evaporated. The crude product was purified by silica gel column chromatography (chloroform as eluent) to yield pure ethyl 10,11-dihydropyrrolo [1,2-b][1,2,5] benzothiadiazepine-11-carboxylate 5,5-dioxide (16) (5,2 g, 41%), melting point 131-134°C after re-crystallization from toluene/ligroin) (121-122°C reported by Stefancich G, et al, 1994).

IR: n 1720, 3360 cam⁻¹. ¹H NMR (CDCl₃) d 1,33 (t, 3H), 4,40 (q, 2H), 5,60 (s br, 1 H), 5,93 (m, 1 H), 6.13-6.30 (m, 2H), 6.58-6.86 (m, 2H), 7.20-7.43 (m, 2H), 7.73-7.93 ppm (m, 1 H).

The reaction is illustrated by the following Scheme 1:

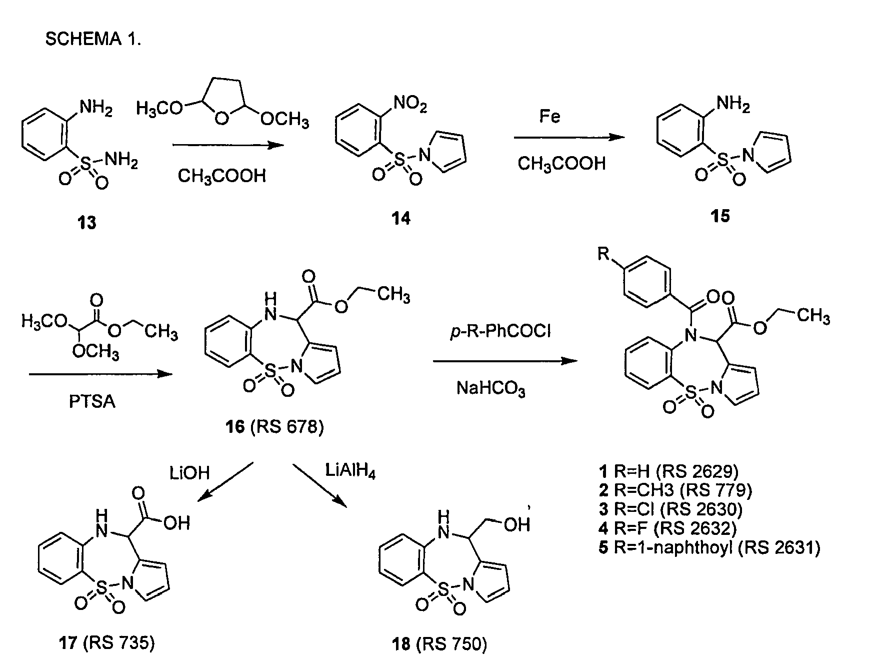

The aroylation of ethyl 10,11-dihydropyrrolo [1,2-b][1,2,5] benzothiadiazepine-11-carboxylate 5,5-dioxide by heating with acyl chlorides acids in boiling 1-bromo-3-chloropropane in the presence of sodium bicarbonate yielded RS-2629, RS-779, RS 2630, RS-2631, RS-2632 compounds (for example ethyl 10,11-dihydro-10- (4-methylbenzoyl) pyrrolo [1,2-b] [1,2,5] benzothiadiazepine-11-carboxylate 5,5-dioxide (RS-779)).

Hydrolysis with lithium hydroxide of (16) at room temperature yielded the correspondent 10,11-dihydropyrrolo [1,2-b][1,2,5] benzothiadiazepine-11-carboxylic 5,5-dioxide acid (RS-735) (17). The reduction with lithium aluminum hydride yielded 10,11-dihydro-11-hydroxymethylpyrrolo [1,2-b][1,2,5] benzothiadiazepine 5,5-dioxide (RS-750) (18) (Scheme 1).

The reaction of 1-[(2-aminophenyl) sulfonil]-1H-pyrrolo (15) with ethyl 3,3-dietoxy acetate in glacial acetic acid has provided ethyl 10,11-dihydropyrrolo [1,2-b] [1,2,5] benzothiadiazepine-11-acetate 5,5-dioxide (19). The aroylation of (19) in the presence od triisobutylamine yielded RS-2645, RS-769, RS-2660, RS-2652, RS-2661 compounds (for example ethyl 10,11-dihydro-10- (4-tolyl) pyrrolo [1,2-b]1,2,5] benzodiazepine-11-acetate 5,5-dioxide (RS-769) (7)). The reaction is illustrated in the following Scheme 2:

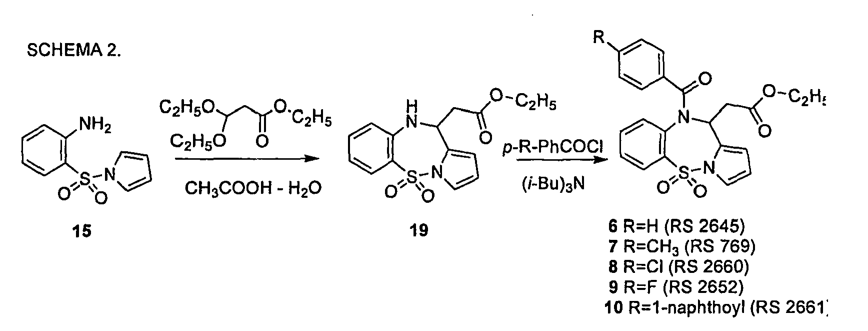

By reaction of N-(2-amminophenyl)-N-methyl-1H-pyrrol-1-ylamine with metoxycarbonil acetyl chloride in anhydrous THF in the presence of triethylamine N-(2-metoxycabonylacetamido)-N-methyl-1H-pyrrol-1-ilamine was obtained. Intramolecular cyclization of this amine with phosphorus oxychloride in dichloromethane yielded methyl 5-methyl-11-metoxycarbonilmethylene-5H,10H-pyrrolo [1,2-b][1,2,5] benzotriazepine which was treated with sodium cyanoborohydride to yield methyl 10,11-dihydro-5-methyl-5H-pyrrol [1,2-b][1,2,5] benzotriazepine-11-acetate RS-20 (20). The aroylaton of (20) for example with benzoyl chloride or 4-metoxybenzoyl chloride yielded respective methyl 10,11-dihydro-5-methyl-10-benzoyl-5H-pyrrolo [1,2-b][1,2,5]benzotriazepine-11-acetate (RS-337) (11) or methyl 10,11-dihydro-5-methyl-10- (4-metoxybenzoyl)-5H-pyrrolo [1,2-b][1,2,5] benzotriazepine-11-acetate (RS-421) (12). The reaction is illustrated in the following Scheme 3:

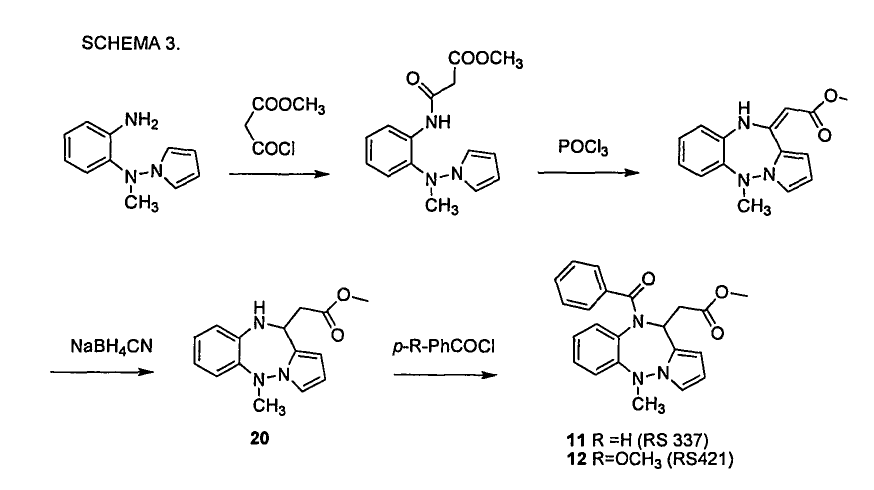

### Preparation of ethyl 10,11-dihydro-10 (benzoyl) pyrrolo [1,2-b] [1,2,5] benzothiadiazepine-11-carboxylate 5,5-dioxide (RS-2629) (1).

A mixture of (16) (1 g, 0,0033 mol), 4-methylbenzoyl chloride (0,52 g, 0,0034 mol), sodium bicarbonate (0.31 g, 0,0037 mol) and 1-bromo-3-chloropropane (50 ml) was reflux heated midnight. After cooling, the mixture was filtered and the solvent evaporated to give a residue which was purified by silica gel column chromatography (chloroform as eluent). Compound (16) was obtained with 40% yield, melting point 172-180°C after re-crystallization from ethanol.

¹H NMR (DMSO-d₆): d 1,30 (t, 7,0 J = Hz, 3H), 4,28 (m, 1H), 4,44 (m, 1 H), 6,16 (m, 1 H), 6,28 (br, 1 H), 6,34 (m, 1H), 7,06 (d, 6,9 J = Hz, 1H), 7.25-7.51 (m, 8H), 7,91 ppm (dd, 7,4 J = 1,5 and Hz, 1 H). IR (neat): n 1185, 1650, 1745 cm⁻¹.

### Preparation of 10,11-dihydropyrrolo [1,2-b][1,2,5] benzothiadiazepine-11-carboxylic -5,5 dioxide acid (RS-735) (17)

A mixture of (16) (1 g, 0,0034 mol), lithium hydroxide 1 N (4 ml), ethanol (10 ml) and THF (10 ml) was stirred at room temperature for 4 hours. The mixture was poured over ice and acidified to pH 2 with hydrochloric acid 1 N. After extraction with ethyl acetate, the organic layer was washed with sodium chloride saturated solution, dried over anhydrous sodium sulfate and the solvent evaporated. The crude product was crushed with toluene and filtered to give (17) (0,9 g, 95%), melting point 162-164°C after re-crystallization from toluene. (159-162°C reported by Stefancich G, et al, 1994).

IR: n 1720, 3330 cm⁻¹. ¹H NMR (DMSO-d₆) d 5,80 (d, 1H), 6.20-6.43 (m, 2H), 6.63-6.86 (m, 1H), 7.06-7.53 (m, 4H), 7.60-7.76 ppm (m, 1 H).

### Preparation of 10,11-Dihydro-11-hydroxymethylpyrrolo [1.2-b] [1.2.5] benzothiadiazepine 5.5-dioxide (RS-750) (18)

A solution of (16) (15 g, 0,049 mol) in anhydrous tetrahydrofuran (150 ml) was slowly added to a suspension of lithium aluminum hydride (1,85 g, 0,049 mol) in same solvent (150 ml) with careful stirring and cooling in an ice-bath. The mixture was stirred at 0°C for 45 minutes and then cautiously treated with crushed ice. After filtration, the solution was concentrated to small volume and extracted with chloroform. The organic layer was washed with sodium chloride saturated solution, dried over anhydrous sodium sulfate and the solvent evaporated. The crude product was purified by silica gel column (chloroform as eluent) to give (18) pure with 65% yield, melting point 135-136°C, after crystallization from toluene/ligroin).

IR: n 3360, 3520 cm⁻¹. ¹H NMR (DMSO-d₆), □ □̅□ (d, 2H), 5.25 (m, 2H), 6,28 (m, 2H), 6,68 (m, 1H), 6.95-7.20 (m, 2H), 7.30-7.55 (m, 2H), 7,68 ppm (dd, 1 H). (Artico M, et al, 1996).

### Preparation of ethyl 10,11-dihyrdrodropyrrolo [1,2-b][1,2,5] benzothiadiazepine-11-acetate 5,5-dioxide (RS-752) (19)

The compound was prepared as (16) employing ethyl 3,3-dietoxypropionate. The yield was 39%, and melting point 118-119°C after re-crystallization from benzene/petroleum ether.

¹H NMR (CDCl₃) δ 1,30 (t, 3H), 3,03 (m, 2H), 4,32 (q, 2H), 5,50 (δ, 1H), 5,75 (m, 1H), 6,10 (m, 1H), 6,22 (t, 1H), 6.61-6.74 (m, 2H), 7.21-7.30 (m, 2H), 7,77 ppm (dd, 1H). IR: n 1730, 3380 cm⁻¹. (Artico M, et al, 1996).

### Preparation of ethyl 10,11-dihdro-10-(benzol)pyrrolo [1,2-b] [1,2,5] benzothiadiazepine-11-acetate 5,5-dioxide (RS-2645) (6)

A mixture of (19) (1 g, 0.0031 mol), benzoyl chloride (0,56 g, 0,0040 mol), triisobutylamine (0,74, 0,0040 mol) and anhydrous dioxane (50 ml) was reflux heated for 48 hours. After reduction to small volume, the mixture was extracted with ethyl acetate, washed with hydrochloric acid 1 N, sodium chloride saturated solution, dried over anhydrous sodium sulfate. After evaporation of the solvent the crude product was purified by silica gel column (dichloromethane as eluent) to give (6) (0,46 g, 35%), melting point 178-181°C after re-crystallization from ethanol.

¹H NMR (DMSO-d₆): δ 1.19 (t, 7,1 J = Hz, 3H), 2 81 (m, 1 H), 2. 91 (m, 1H), 4.12 (m, 2H), 6.35-6.40 (m, 2H), 6.56 (br, 1H), 7.22-7.58 (m, 9H), 8.00 ppm (m, 1H). IR (neat): n 1186,1315,1655,1725 cm⁻¹.

### Preparation of methyl 10.11-dihydro-5-methyl-10-benzoyl-5H-pyrrolo [1,2-b] [1,2,5] benzotriazepine-11-acetate (RS-337) (11)

A solution of benzoyl chloride (0,0083 mol) in anhydrous tetrahydrofuran (50 ml) was added in drops to a solution of (20) (2,25 g, 0,0083 mol) and triethylamine (0,84 g, 0,0083 mol) in the same solvent (50 ml) by cooling in an ice-bath. The mixture was reflux heated for 72 hours, filtered and the residue was purified by silica gel column (dichloromethane as eluent) to give (11), 90% yield, melting point 88-89°C after re-crystallization from benzene/petroleum ether.

¹H NMR (90 MHz) (CDCl₃): δ 2.77 (δ, 7,0 J = Hz, 2H), 3.42 (s, 3H), 3.70 (s, 3H), 5.93 (m, 2H), 6.52 (t, 7,0 J = Hz, 1H), 6.87-7.47 ppm (m, 10H). Anal. Calcd. for C₂₂H₂₁N₃O₃ (375,42) (Stefancich G, et al, 1990).

By this procedure (RS-421) (12) compound was prepared using 4-metoxybenzoyl chloride.

### EXAMPLE 2: In vitro studies on K562, HL60, Jurkat, U937, Glivec resistant K562 leukemic cell lines treated with RS678 and RS779 compounds

### MATERIALS AND METHODS

The used cellular lines are: K562 (human erythroleucemic cell line), HL60 (human acute promyelocytic leukemia cell line), Jurkat (cell line from acute lymphoblastic T variant leukemias), U937 (acute promyelocitic leukemia cell line) and Glivec resistant K562 (this cell line was supplied kindly by Prof. Carlo Gambacorti-Passerini of the Dipartimento of Clinica Medica, Università of Milan-Bicocca, Monza, Italy) (Gambacorti-Passerini CB, et al, 2002).

The cell lines were incubated with RS678 and RS779 synthetic compounds at 10 µM concentration for 8, 16 and 24 hours, subsequently washed with PBS and 2,5 mg/ml of propidium iodide. This phenanthridine dye, when intercalated into double-stranded DNA forms a sufficiently stable complex that under excitation emits a red fluorescent light. By means of this coloration it is possible to discriminate in an heterogenous population cells with different DNA contents and therefore to estimate the distribution of the cellular populations based on their DNA contents and whether possibly, as a result from pharmacological treatments, alterations at level of cell cycle have manifested. The labeled cell lines were analyzed using flow FACSort cytofluorimeter.

### RESULTS

In the earlier step of the study, experiments *in vitro* on five different hematological cell lines were carried out. The two compounds are able to induce apoptosis after 8 hours in all the tested different leukemic cell line types. Moreover, the apoptotic phenomenon in the Glivec resistant K562 cell line occurred 16 hours after the treatment.

While control cells have the classic DNA distribution, modifying the cell growth conditions (a different leukemic cell model), i. e. by adding a proapoptotic agent (RS678 and RS 779) an increase in the percentage of the apoptotic cells in comparison with non treated cells is observed (Figures 7, 8 and 9).

The CML results from Philadelphia chromosome, an anomaly that produces carcinogenic protein called BCR-ABL. Glivec acts by binding BCR-ABL and completely blocking activity thereof, arresting in such a way the tumour progress. However, subsequently, a significant number of patients developed a resistance as cancer cells have been mutated and adapted. Now new drugs are being sought for in order to prevent the resistance to Glivec: in the experiments carried out on the resistant cell line, apoptosis, assessed using propidium iodide, was obtained, therefore it can be assumed that the two used synthetic compounds induce the cellular death of the Glivec resistant known mutants.

### EXAMPLE 3: In vitro studies on K562 cell lines treated with pyrrolo [1,2-b] [1,2,5] benzothiadiazepine (PBTD) and pyrrolo [1,2-b] [1,2,5] benzotriazepine

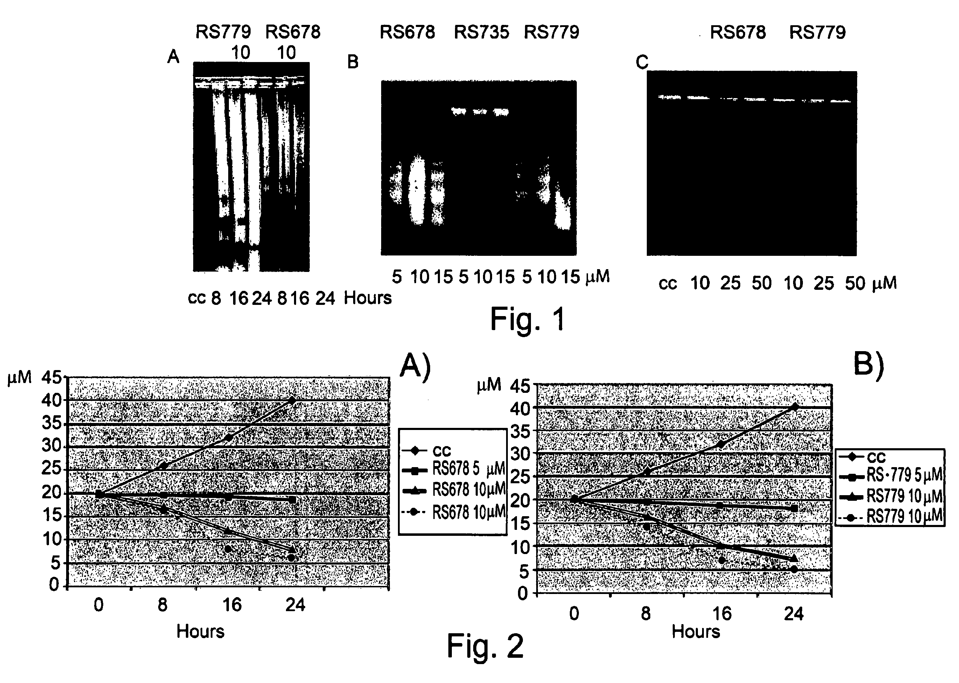

Three compounds of the PBTD (RS-678, RS-735 and RS-779) series were tested on K562 cell line and it was assessed that only two (RS-678 and RS-779) are able to induce apoptosis. Samples were tested using the two different synthetic compounds for 8, 16 and 24 hours at different concentration (5 µM, 10 µM, 15 µM) and subsequently, by means of molecular biology techniques, the DNA fragmentation, that is the condensation and cutting of the chromatin in 180-200 base pair fragments or entire multiples of these numbers, length corresponding to that of internucleosomal DNA stretches (Figure 1 panels A e B).

From experiments a 10 µM concentration was selected as 15 µM one activated the inhibiting effect on the cellular proliferation (Figure 2, panels A e B).

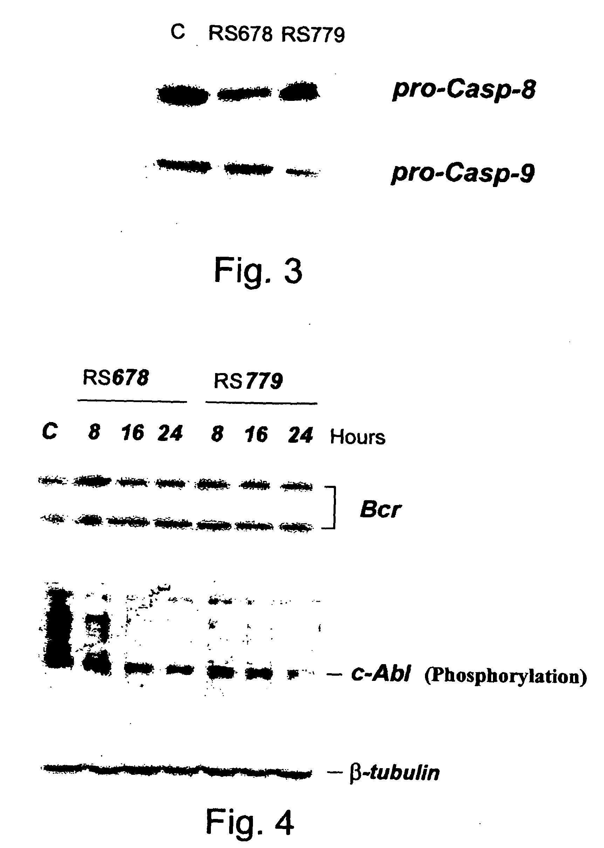

Moreover, in order to elucidate the apoptotic pathway triggered by these two substances, Western Blotting experiments and tyrosine-kinase assays were carried out and evidenced that:
(i) these compounds activate specific molecules involved in the apoptotic process (caspase 9 and caspase 8) (Figure 3);
(II) they work by triggering an alternative pathway with respect to existing drugs (anti-tyrosine-kinase drugs) because the down-regulation of the bcr-abl protein occurs 16 hours after treatment and changes in the phosphorylation profile of the aforesaid protein did not occur, suggesting that its down-regulation is not involved in the early steps of the cell dearth process (Figure 4).

In order to elucidate the mechanism triggered by the compounds, the latter were tested on R2C cell line, derived from rat Leydig tumour: this cell line shows several benzodiazepine receptors of peripheral type. Two compounds were tested at concentrations both 10 µM and 50 µM for 8, 16 and but DNA fragmentation was not detected, whereby it is possible to assume that these compounds do not bound to the aforesaid receptor (Figure 1 C).

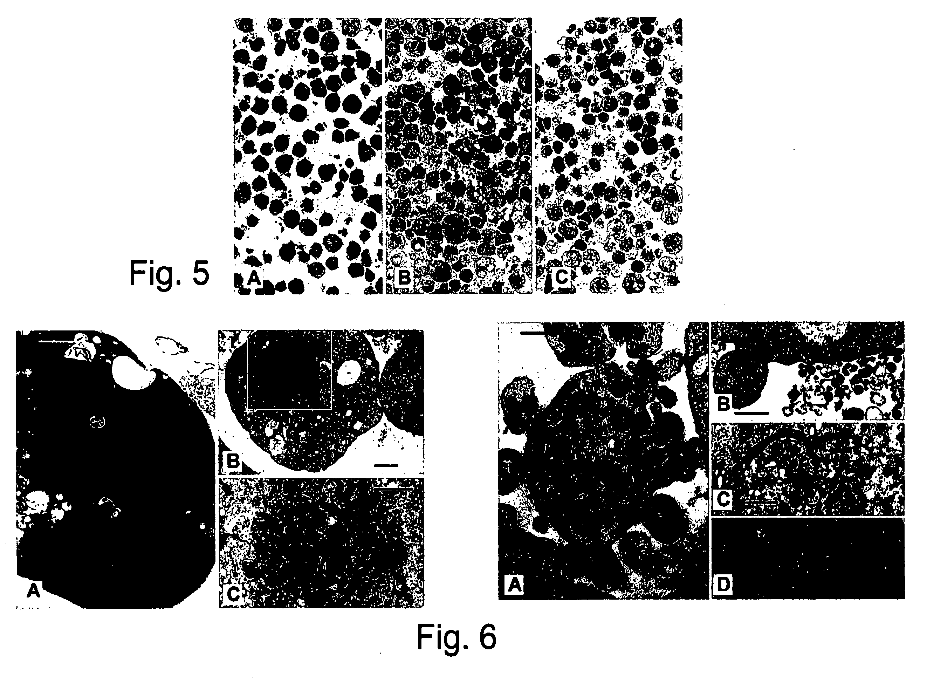

Experiments with both optical and electronic microscopy were carried out: Figure 5 shows typical apoptotic morphology, cytoplasmic vacuolization, chromatin condensation; Figure 6 shows the formation of the membrane enveloped nuclear bodies and cytoplasmic vacuolization

In a second experimental step *in vitro* were tested two synthetic compounds (RS-678 and RS-779) on mixed limphocytic cultures from marrow or peripheral blood of patients suffering from chronic myelogenous leukemia at onset, some of which, treated with GLIVEC, which they then became resistant to, subsequently have been subjected to new experimental protocol with anti-tyrosine-kinase BMS-354825 (samples were withdrawn after patient consensus to the experimentation) in order to reach the attenuation of the clinical symptomatology.

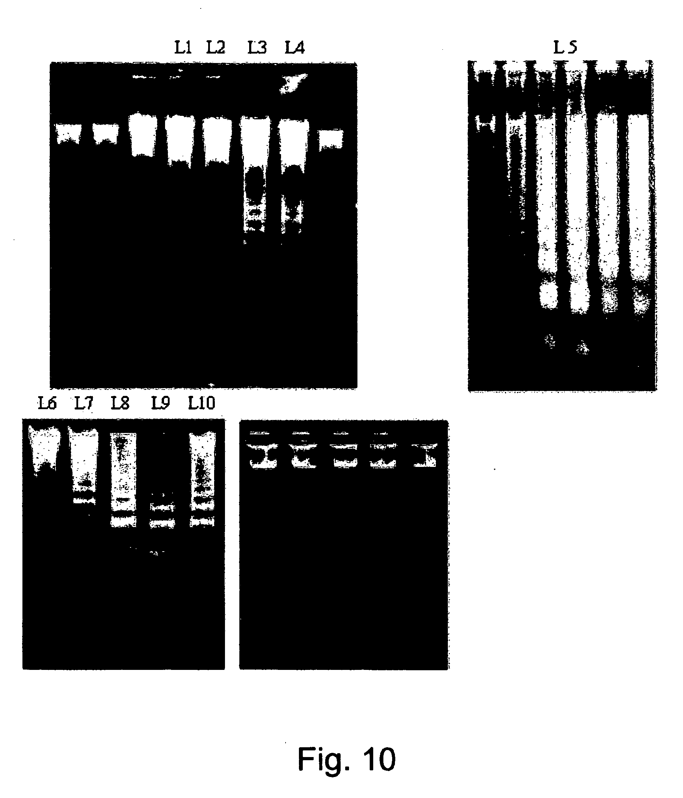

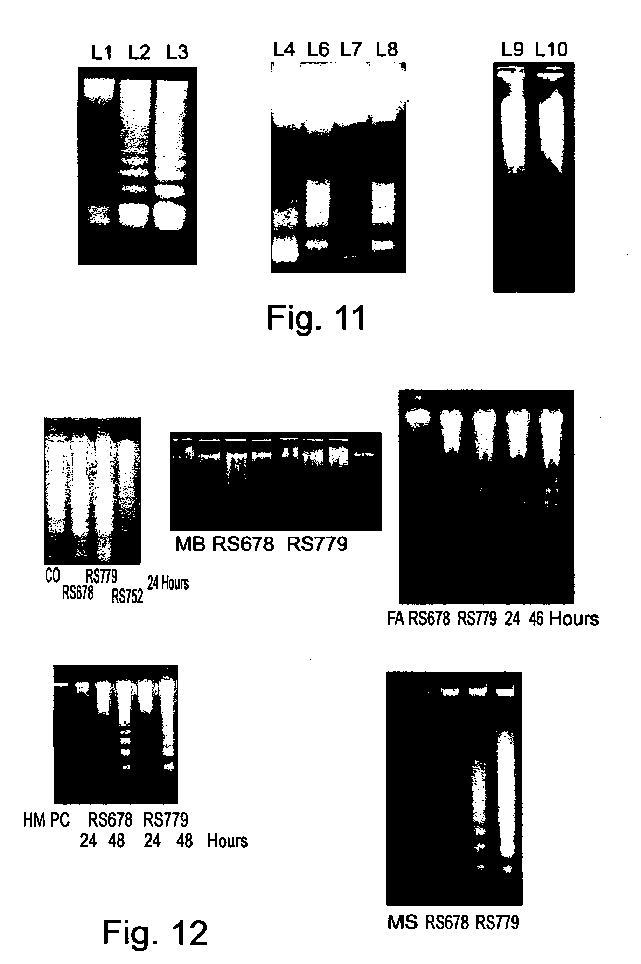

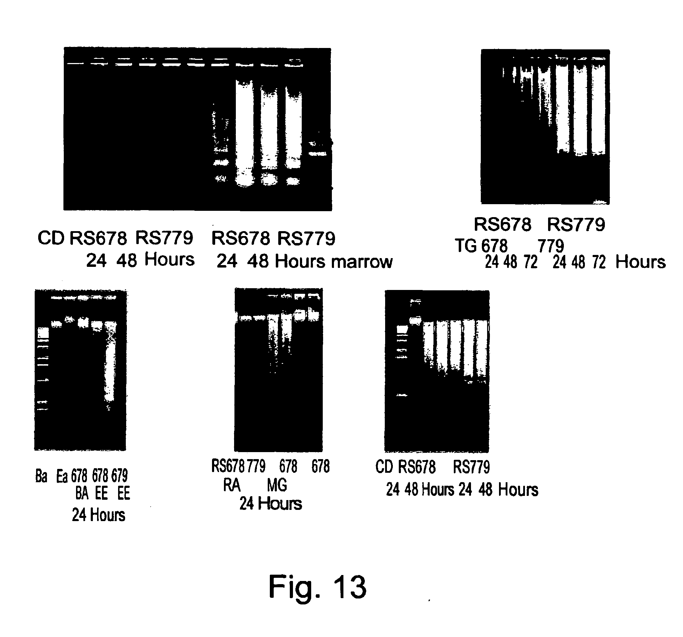

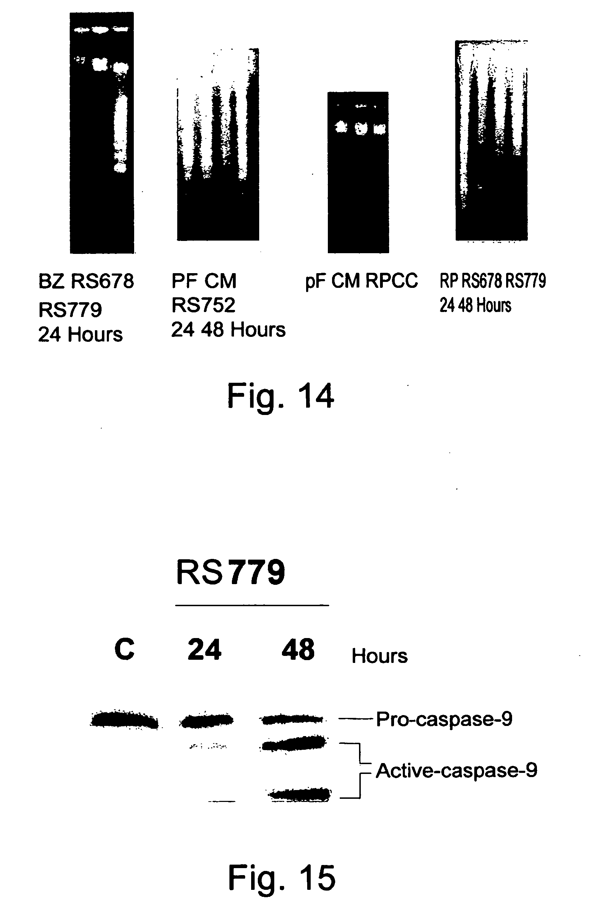

Mixed lymphocitic cultures were incubated with RS-678 and RS -779 for 8, 16, 24 and 48 hours at 10 µM concentration and in any case we obtained the DNA fragmentation (Figure 10, Figure 11, Figure 12, Figure 13, Figure 14). Moreover on a sample by Western Blotting technique a strong activation of caspase 9 was detected (Figure 15).

The evidence that the same compounds are active also for patients in blast crisis of disease, evolved in chronic phase after treatment with Glivec and therefore with a more aggressive disease, enhances the antiapoptotic action thereof and confirms the potential use for the treatment of this pathology.

EXAMPLE 4: *Studies in vitro on ovary OVCAR3, and ARO and NPA thyroid carcinoma cell lines treated with pyrrolo [1,2-b] [1,2,5] benzothiadiazepine (PBTD) and pyrrolo [1,2-b] [1,2,5] benzotriazepine compounds*

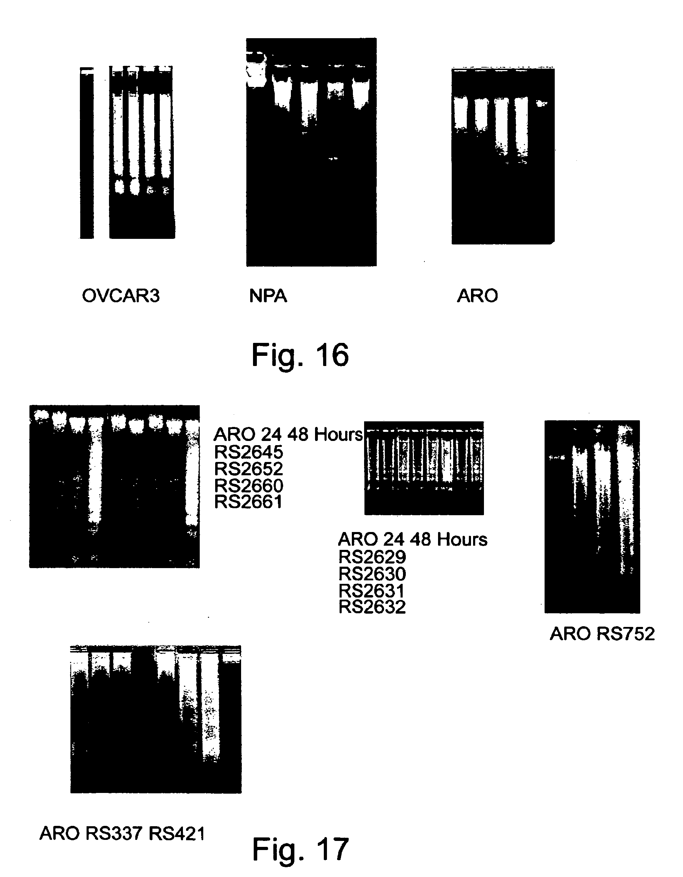

The authors also have tested compounds according to the invention, in particular RS-678 and RS-779 compounds, also on OVCAR3 ovary carcinoma cell lines, ARO anaplastic thyroid and NPA papillary thyroid human carcinoma cell lines. The concentration was 10 µM for 24 and 48 hours, and also in this case induction of the DNA fragmentation was obtained (Figure 16).

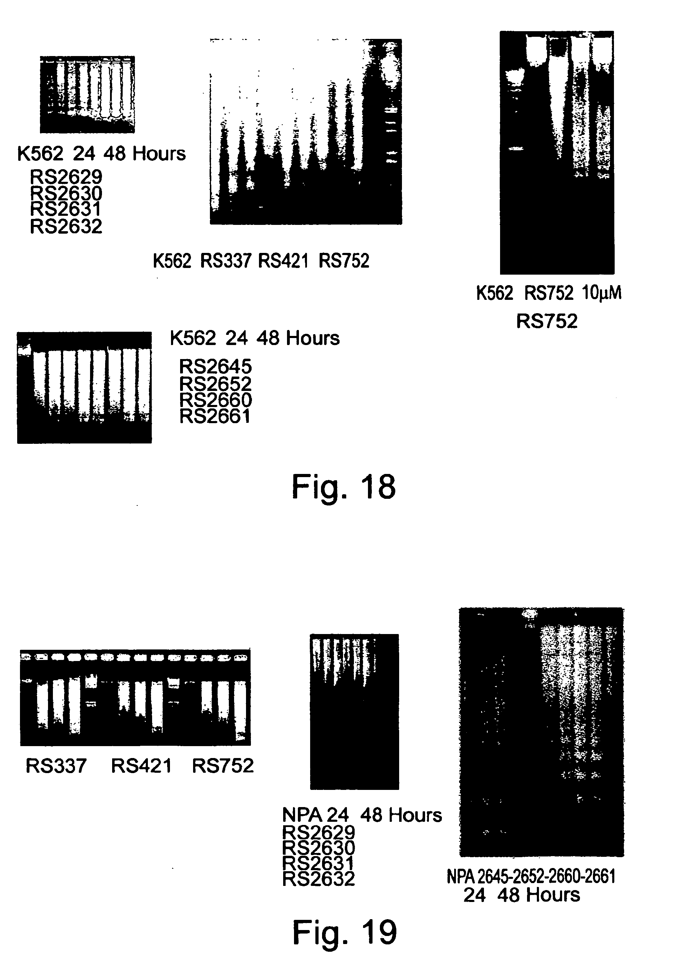

Subsequently, the same cell lines, except OVCAR3, were tested with fourteen new synthetic compounds at 10 µM concentration for 24 and 48 hours: eleven induced the expected cellular death. Such phenomenon was assessed by means of DNA fragmentation on agarose gel (Figures 17-21).

Moreover, it was detected that for the tested cell lines some synthetic compounds like 2629, 2630, 2645 induce greater cellular death, that is the percentage of dead cells for 2629 and 2630 compounds for ARO cell line is approximately 60% after 24 hours, while for RS-779 compound is 45% after 24 hours; for NPA and K562 cell lines the percentage of apoptosis after 24 hours is 80% and 85% with compounds 2629 and 2630, respectively; for RS-779 compound is 50% (NPA) and 70% (K562) after 24 hours.

Experiments with gastric (GEO) and prostate (PC3) carcinoma cell lines, both resistant to EGFR inhibitors like C225 (cetuximab), a monoclonal chimeric human and murine anti-EGFR and ZD1839 (gefitinib) antibody, a small EGFR tyrosine-kinase domain selective inhibiting molecule, are in progress (Tuccillo C, et al, 2005; Tortora G, et al, 2004; Di Lorenzo G, et al, 2005; Ciardiello F, et al, 2004).

It is apparent from the above reported experiments that the action mechanism of PBTD suitable to induce apoptosis in various tumour cell lines (wide spectrum of action) seems to be independent from the activation profile of the cellular tyrosine-kinase, targets of inhibiting drugs; the aim of the therapy with PBTD would be therefore to prevent the occurrence of clones selected for the resistance to protein kinase inhibition.

### BIBLIOGRAPHY

- O'Hare T, Walters DK, Stoffregen EP, Jia T, Manley PW, Mestan J, Cowan-Jacob SW, Lee FY, Heinrich MC, Deininger MW, Druker BJ. Cancer Res. 2005; 65(11):4500-5.
- Burgess MR, Skaggs BJ, Shah NP, Lee FY, Sawyers CL. Proc Natl Acad Sci USA. 2005 1;102(9):3395-400.
- Sawyers CL. N Eng J Med. 1999; 340:1330-1340.
- Lugo TG, Pendergast AM, Muller AJ, Witte ON. Science. 1990; 247:1079-1082.
- Daley GQ, Van Etten RA, Baltimore D. Science. 1990; 247:824-830.
- Cortes J, O'Brien S, Kantarjian H. Blood. 2004;104(7):2204-2205.
- Hochhaus A, La Rosée P. Leukemia 2004; 18;1321-1331.
- Weisberg E, Griffin JD. Blood. 2000; 95: 3498-3505.
- Stefancich G, Silvestri R, Pagnozzi E, Artico MJ. Heterocyclic Chem. 1994; 31: 867-869.
- Artico M, Silvestri R, Pagnozzi E, Stefancich G, Massa S, Loi A. G, Scano P, Corrias S, Spiga MG, La Colla, P. Bioorg & Med. Chem. 1996, 4, 837-850.
- Artico M, Silvestri R, Pagnozzi E, Stefancich G, Massa S, La Colla P. II Farmaco 1996; 51: 425-430.
- Stefancich G, Artico M, Silvestri R, Prosini PP. Farmaco 1990; 45: 817-831.
- Mahon F.X., Deininger M.W., Schultheis B., et al. Blood. 2000, 96: 1070-1079.
- Bhatia R, Holtz M, Niu N, et al. Blood 2003; 101:4701-07.
- Goldman JM, Melo JV. N Engl J Med 2003; 349: 1451-1464.
- Ottmann OG, Druker BJ, Sawyers CL, Goldman JM, Reiffers J, Silver RT et al. Blood 2002; 100: 1965-1971.
- Lydon NB, Druker BJ. Leukemia Res 2004; 28S1: S29-S38.
- Buchdunger E, Cioffi CL, Law N, Stover D, Ohno-Jones S, Druker BJ et al. J Pharmacol Exp Ther 2000; 295: 139-145.
- Okuda K, Weisberg E, Gilliland DG, Griffen JD. Blood 2001; 97: 2440-2448.
- Sawyers CL, Hochhaus A, Feldman E, Goldman JM, Miller CB, Ottmann OG et al. Blood 2002; 99: 3530-3539.
- Talpaz M, Silver RT, Druker BJ, Goldman JM, Gambacorti-Passerini C, Guilhot F et al. Blood 2002; 99: 1928-1937.
- O'Brien SG, Guilhot F, Larson RA, Gathmann I, Baccarani M, Cervantes F et al. N Engl J Med 2003; 348: 994-1004.
- Kantarjian H, Sawyers C, Hochhaus A, Guilhot F, Schiffer C, Gambacorti-Passerini C et al. N Engl J Med, 2002; 346: 645-652.
- Druker, B. J. Oncogene 2002; 21: 8541-8546.
- Druker, B. J., Sawyers, C. L., Kantarjian, H., Resta, D. J., Reese, S. F., Ford, J. M., Capdeville, R. & Talpaz, M. N. Engl. J. Med., 2001; 344:1038-1042
- Kantarjian H, Sawyers C, Hochhaus A, Guilhot F, Schiffer C, Gambacorti-Passerini C, Niederwieser D, Resta D, Capdeville R, Zoellner U, Talpaz M, Druker B, Goldman J, O'Brien SG, Russell N, Fischer T, Ottmann O, Cony-Makhoul P, Facon T, Stone R, Miller C, Tallman M, Brown R, Schuster M, Loughran T, Gratwohl A, Mandelli F, Saglio G, Lazzarino M, Russo D, Baccarani M, Morra E. N. Engl. J. Med., 2002; 346: 645-52.
- O'Brien SG, Guilhot F, Larson RA, Gathmann I, Baccarani M, Cervantes F, Cornelissen JJ, Fischer T, Hochhaus A, Hughes T, Lechner K, Nielsen JL, Rousselot P, Reiffers J, Saglio G, Shepherd J, Simonsson B, Gratwohl A, Goldman JM, Kantarjian H, Taylor K, Verhoef G, Bolton AE, Capdeville R, Druker BJ; N. Engl. J. Med., 2003; 348: 994-1004.
- Wu CJ, Neuberg D, Chillemi A, McLaughlin S, Hochberg EP, Galinsky I, DeAngelo D, Soiffer RJ, Alyea EP, Capdeville R, Stone RM, Ritz J. Leuk. Lymphoma, 2002; 43:2281-2289.
- Azam, M., Latek, R. R. & Daley, G. Q. Cell, 2003; 112:831-843.
- Grieco M, Santoro M, Berlingieri MT, Melillo RM, Donghi R, Bongarzone I, Pierotti MA, Della Porta G, Fusco A, Vecchio G. Cell, 1990; 60:557-563.
- Pierotti MA, Bongarzone I, Borello MG, Greco A, Pilotti S, Sozzi G. Genes Chromosomes Cancer, 1996; 16:1-14.
- Santoro M, Dathan NA, Berlingieri MT, Bongarzone I, Paulin C, Grieco M, Pierotti MA, Vecchio G, Fusco A. Oncogene, 1994; 9:509-516.
- Morotti A, Cilloni D, Messa F, Arruga F, Defilippi I, Carturan S, Catalano R, Rosso V, Chiarenza A, Pilatrino C, Guerrasio A, Taulli R, Bracco E, Pautasso M, Baraban D, Gottardi E, Saglio G. Valproate enhances imatinib-induced growth arrest and apoptosis in chronic myelogenous leukemia cells. Cancer. 2006 Mar 1;106(5):1188-96.
- Tuccillo C, Romano M, Troiani T, Martinelli E, Morgillo F, De Vita F, Bianco R, Fontanini G, Bianco RA, Tortora G, Ciardiello F. Clin Cancer Res. 2005; 11(3):1268-76.
- Tortora G, Bianco R, Daniele G. J Chaemother. 2004; 16 Suppl 4:41-3.
- Di Lorenzo G, De Placido S, Autorino R, De Laurentiis M, Mignogna C, D'Armiento M, Tortora G, De Rosa G, D'Armiento M, De Sio M, Bianco AR, D'Armiento FP. Prostate Cancer Prostatic Dis. 2005; 8(1):54-9.
- Ciardiello F, Bianco R, Caputo R, Caputo R, Damiano V, Troiani T, Melisi D, De Vita F, De Placido S, Bianco AR, Tortora G. Clin Cancer Res. 2004; 10(2):784-93.
- Gambacorti-Passerini CB, Rossi F, Verga M, Ruchatz H, Gunby R, Frapolli R, Zucchetti M, Scapozza L, Bungaro S, Tornaghi L, Rossi F, Pioltelli P, Pogliani E, D'Incalci M, Corneo G. Differences between in vivo and in vitro sensitivity to imatinib of Bcr/Abl+ cells obtained from leukemic patients. Blood Cells Mol Dis. 2002; 28(3):361-72.
- US 4444688;
- US 4062852;
- GB 1476684
- US 3506646
- WO 03091232
- US 3453266
- JP 2138272
- WO 2004/069843
- US 6156746
- US 3506646

## Claims

1. Use of derivative compounds of benzodiazepines of formula (i): 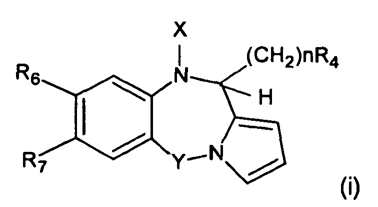 wherein
Y it is SO₂ or NR, wherein R is H or C₁-C₆ alkyl;
X is H, C₁-C₁₂ alkyl, -CO, -SO, -SO₂, or - CO-R₂, SO-R₂, SO₂-R₂, wherein R₂ is selected from H, C₁-C₁₂ alkyl, (C₃-C₈ cycloalkyl) C₀-C₆ alkyl, C₂-C₆ alkenyl, C₂-C₆ alkynyl, C₂-C₆ alkoxycarbonyl, phenyl, benzyl, naphtyl, biphenyl, or heterocycle, each para-, meta- or ortho-substituted independently of each other with 0 to 3 substituents selected from halogen, -CN, -NH₂, -OH, -NO₂, COOR₃, wherein R₃ is selected from H, C₁-C₁₂ alkyl, (C₃-C₈ cycloalkyl) C₀-C₆ alkyl, C₁-C₆ alkoxy, C₂-C₆ alkenyl, C₂-C₆ alkynyl, or C₂-C₆ alkoxy carbonyl; phenyl, benzyl, naphtyl, biphenyl, or heterocycle,
n is 0-6;
R₄ is selected from H, OH, COOH, CN, C₁-C₆ alkyl, C₁-C₆ alkoxy, C₂-C₆ alkenyl, C₂-C₆ alkynyl, C₂-C₆ alkoxycarbonyl, -CO, -SO,-SO₂, or -CO-R₅, SO-R₅, SO₂-R_{2,} -OCO-R₅, OSO-R₅, OSO₂-R₅, COOR₅, SOOR₅, SO₂OR₅, NHR₅, NHCOR₅, NHSO₂R₅, SR₅, SCOR₅, wherein R₅ is selected from H, C₁-C₆ alkyl, C₁-C₆ alkoxy, C₂-C₆ alkenyl, C₂-C₆ alkynyl, or C₂-C₆ alkoxycarbonyl, phenyl, benzyl, naphtyl, biphenyl, or heterocycle, each para-, meta- or ortho-substituted independently of each other with 0 to 3 substituents selected from halogen, -CN, -NH₂, -OH, -NO₂, COOR₅ wherein R₅ is selected from H, C₁-C₁₂ alkyl, (C₃-C₈ cycloalkyl) C₀-C₆ alkyl, phenyl, benzyl, naphtyl, biphenyl, or heterocycle, C₁-C₆ alkyl, C₁-C₆ alkoxy, C₂-C₆ alkenyl, C₂-C₆ alkynyl, or C₂-C₆ alkoxy carbonyl;
R₆ and R₇ independently of each other are selected from the group consisting of H, OH, halogen, CN, NH₂, NO₂, COOR₃, C₁-C₆ alkyl, C₁-C₆ alkoxy, C₂-C₆ alkenyl, C₂-C₆ alkynyl, and C₂-C₆ alkoxy carbonyl;
for the preparation of a medicament for the treatment of solid or liquid tumours.

2. Use according to claim 1, wherein n is 0 and R₄ is COOR₅, wherein R₅ is as defined in claim 1.

3. Use according to claim 1, wherein n is 0 or 1 and R₄ is COOH.

4. Use according to claim 1, wherein n is 1 and R₄ is COOR₅, wherein R₅ is as defined in claim 1.

5. Use according to claim 1, wherein n is 1 and R₄ is OH.

6. Use according to claim 1, wherein n is 1 and R₄ is 4-chlorophenyl carboniloxy.

7. Use according to claim 2 or 4, wherein R₅ is C₁-C₆ alkyl, preferably methyl or ethyl.

8. Use according to any of preceding claims wherein X is H.

9. Use according to any of claims 1 to 7 wherein X is -CO-R₂, wherein R₂ is as defined in claim 1.

10. Use according to claim 9, wherein R₂ is phenyl or naphtyl.

11. Use according to claim 9, wherein R₂ is phenyl monosubstituted in para position with CI, F, OCH₃, or CH₃.

12. Use according to any of claims 1 to 11 wherein R₆ and R₇ are H.

13. Use according to any of claims 2 to 12 wherein Y is SO₂

14. Use according to claim 13, said compounds being selected from the group consisting of:
ethyl 10,11-dihydropyrrolo [1,2-b] [1,2,5] benzo-thiadiazepine-11-carboxylate 5,5-dioxide (RS 678);
ethyl 10-(4-tolyl)-10,11-dihydropyrrolo [1,2-b][1,2,5] benzo thiadiazepine-11-acetate 5,5-dioxide (RS-779);
10,11-dihydropyrrolo [1,2-b] [1,2,5] benzothiadiazepine-11-carboxylic 5,5-dioxide acid (RS-735);
10,11-dihydropyrrolo [1,2-b] [1,2,5] benzothiadiazepine-11-acetic 5,5-dioxide acid (RS-791);
10,11-dihydropyrrolo [1,2-b] [1,2,5] benzothiadiazepine-11-methanol 5,5-dioxide (RS-750);
ethyl 10,11-dihydro-10-(4-tolyl) pyrrolo [1,2-b] [1,2,5]-benzothiadiazepine acetate 5,5-dioxide (RS-769);
ethyl 10,11-dihydro-10-(benzoyl) pyrrolo [1,2-b] [1,2,5] benzothiadiazepine-11-carboxylate 5,5-dioxide (RS-2629);
ethyl 10,11-dihydropyrrolo [1,2-b] [1,2,5] benzothiadiazepine-11-acetate 5,5-dioxide (RS-752);
ethyl 10,11-dihydro-10- (benzoyl) pyrrolo [1,2-b] [1,2,5] benzothiadiazepine-11-acetate 5,5-dioxide (RS-2645);
10,11-dihydropyrrolo [1,2-b] [1,2,5] benzothiadiazepine-11-acetic 5,5-dioxide acid (RS-761);
11-(4-chloro pheny benzoyloxy methylene) 10,11-dihydro pyrrolo [1,2-b] [1,2,5] benzothiadiazepine 5,5-dioxide (RS-786);
ethyl 10,11-dihydro-10- (4-chlorobenzoyl) pyrrolo [1,2-b] [1,2,5] benzothiadiazepine-11-carboxylate 5,5-dioxide (RS-2630);
ethyl 10,11-dihydro-10-(1-naphtoyl) pyrrolo [1,2-b][1,2,5] benzothiadiazepine-11-carboxylate 5,5-dioxide (RS-2631);
ethyl 10,11-dihydro-10- (4-fluorobenzoyl) pyrrolo [1,2-b] [1,2,5] benzothiadiazepine-11-carboxylate 5,5-dioxide (RS-2632);
ethyl 10,11-dihydro-10-(4-chlorobenzoyl) pyrrolo [1,2-b] [1,2,5] benzothiadiazepine-11-acetate 5,5-dioxide (RS-2660);
ethyl 10,11-dihydro-10-(1-naphtoyl) pyrrolo [1,2-b] [1,2,5] benzothiadiazepine-11-acetate 5,5-dioxide (RS-2661);
or
ethyl 10,11-dihydro-10- (4-fluorobenzoyl) pyrrolo [1,2-b] [1,2,5] benzothiadiazepine-11-acetate 5,5-dioxide (RS-2652).

15. Use according to any of claims 2 to 12, wherein Y is NR, and R is CH3.

16. Use according to claim 15, said compounds being selected from the group consisting of :
methyl 10,11-dihydro-5-methyl-5H-pyrrolo[1,2-b][1,2,5] benzo-thiazepin-11-acetate;
methyl 10,11-dihydro-5-methyl-10-benzoyl-5H-pyrrolo[1,2-b] [1,2,5] benzothiazepin-11-acetate (RS-337);
or methyl 10,11-dihydro-5-methyl-10- (4-metoxybenzoyl)-5H-pyrrolo[1,2-b][1,2,5] benzo-thiazepin-11-acetate (RS-421).

17. Use according to anyone of the claims 1-16, wherein said solid tumours are carcinomas selected from the group consisting of ovary, thyroid, colon, pancreas, breast, gastric, prostate, pulmonary carcinomas.

18. Use according to anyone of the claims 1-16, wherein said liquid tumours are selected from leukemias.

19. Use according to claim 18, wherein said leukemia is chronic myelogenous leukemia (CML).

20. Use according to claim 18, wherein said leukemia is acute myelogenous leukemia (AML).

21. Use according to claim 18, wherein said leukemia is acute lymphoblastic leukemia (ALL).

22. Use of a pharmaceutical composition comprising at least one of compounds as defined in any of claims 1 to 16, as active principles, together with one or more pharmacologically acceptable adjuvants and/or excipients for the treatment of solid or liquid tumours.

23. Use according to claim 22, wherein said solid tumours are carcinomas, selected from the group consisting of ovary, thyroid, colon, pancreas, breast, gastric, pulmonary, prostate carcinomas.

24. Use according to claim 22, wherein said liquid tumours are selected from leukemias.

25. Use according to claim 24, wherein said leukemia is chronic myelogenous leukemia (CML).

26. Use according to claim 24, wherein said leukemia is acute myelogenous leukemia (AML).

27. Use according to claim 24, wherein said leukemia is acute lymphoblastic leukemia (ALL).

28. Derivatives compounds of benzodiazepines of formula (i), said compounds being selected from the group consisting of:
ethyl 10,11-dihydro-10-(benzoyl) pyrrolo [1,2-b] [1,2,5] benzothiadiazepine-11-carboxylate 5,5-dioxide (RS-2629);
ethyl 10,11-dihydro-10- (benzoyl) pyrrolo [1,2-b] [1,2,5] benzothiadiazepine-11-acetate 5,5-dioxide (RS-2645);
ethyl 10,11-dihydro-10- (4-chlorobenzoyl) pyrrolo [1,2-b] [1,2,5] benzothiadiazepine-11-carboxylate 5,5-dioxide (RS-2630);
ethyl 10,11-dihydro-10-(1-naphtoyl) pyrrolo [1,2-b][1,2,5] benzothiadiazepine-11-carboxylate 5,5-dioxide (RS-2631);
ethyl 10,11-dihydro-10- (4-fluorobenzoyl) pyrrolo [1,2-b] [1,2,5] benzothiadiazepine-11-carboxylate 5,5-dioxide (RS-2632);
ethyl 10,11-dihydro-10-(4-chlorobenzoyl) pyrrolo [1,2-b] [1,2,5] benzothiadiazepine-11-acetate 5,5-dioxide (RS-2660);
ethyl 10,11-dihydro-10-(1-naphtoyl) pyrrolo [1,2-b] [1,2,5] benzothiadiazepine-11-acetate 5,5-dioxide (RS-2661);
or
ethyl 10,11-dihydro-10- (4-fluorobenzoyl) pyrrolo [1,2-b] [1,2,5] benzothiadiazepine-11-acetate 5,5-dioxide (RS-2652).

## Patentansprüche

1. Verwendung von Derivat-Verbindungen von Benzodiazepinen der Formel (i) 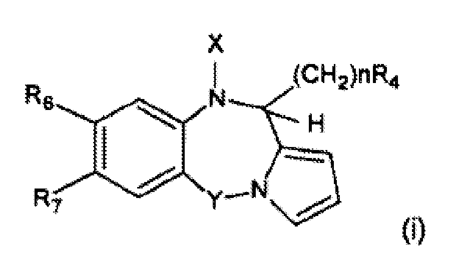 worin
Y SO₂ oder NR, wobei R H oder C₁-C₆-Alkyl ist, darstellt;
X H, C₁-C₁₂-Alkyl, -CO, -SO, -SO₂ oder -CO-R₂, SO-R₂, SO₂-R₂, wobei R₂ ausgewählt ist aus H, C₁-C₁₂-Alkyl, (C₃-C₈-Cycloalkyl)C₀-C₆-alkyl, C₂-C₆-Alkenyl, C₂-C₆-Alkinyl, C₂-C₆-Alkoxycarbonyl, Phenyl, Benzyl, Naphthyl, Biphenyl oder Heterocyclyl, jeweils unabhängig voneinander mit 0 bis 3 Substituenten, ausgewählt aus Halogen, -CN, -NH₂, -OH, -NO₂, COOR₃, wobei R₃ aus H, C₁-C₁₂-Alkyl, (C₃-C₈-Cycloalkyl)C₀-C₆-alkyl, C₁-C₆-Alkoxy, C₂-C₆-Alkenyl, C₂-C₆-Alkinyl oder C₂-C₆-Alkoxycarbonyl ausgewählt ist, para-, meta- oder ortho-substituiert; Phenyl, Benzyl, Naphthyl, Biphenyl oder Heterocyclyl darstellt;
n 0-6 ist;
R₄ ausgewählt ist aus H, OH, COOH, CN, C₁-C₆-Alkyl, C₁-C₆-Alkoxy, C₂-C₆-Alkenyl, C₂-C₆-Alkinyl, C₂-C₆-Alkoxycarbonyl, -CO, -SO, -SO₂, oder -CO-R₅, SO-R₅, SO₂-R₂, -OCO-R₅, OSO-R₅, OSO₂-R₅, COOR₅, SOOR₅, SO₂-OR₅, NHR₅, NHCOR₅, NHSO₂R₅, SR₅, SCOR₅, wobei R₅ ausgewählt ist aus H, C₁-C₆-Alkyl, C₁-C₆-Alkoxy, C₂-C₆-Alkenyl, C₂-C₆-Alkinyl oder C₂-C₆-Alkoxycarbonyl, Phenyl, Benzyl, Naphthyl, Biphenyl oder Heterocyclyl, jeweils unabhängig voneinander mit 0 bis 3 Substituenten, welche ausgewählt sind aus Halogen, -CN, -NH₂, - OH, -NO₂, COOR₅, wobei R₅ aus H, C₁-C₁₂-Alkyl, (C₃-C₈-Cycloalkyl)C₀-C₆-alkyl, Phenyl, Benzyl, Naphthyl, Biphenyl oder Heterocyclyl, C₁-C₆-Alkyl, C₁-C₆-Alkoxy, C₂-C₆-Alkenyl, C₁-C₆-Alkinyl oder C₂-C₆-Alkoxycarbonyl ausgewählt ist, para-, meta- oder ortho-substituiert;
R₆ und R₇ unabhängig voneinander aus der Gruppe ausgewählt sind, welche aus H, OH, Halogen, CN, NH₂, NO₂, COOR₃, C₁-C₆-Alkyl, C₁-C₆-Alkoxy, C₂-C₆-Alkenyl, C₂-C₆-Alkinyl und C₂-C₆-Alkoxycarbonyl besteht;
zur Herstellung eines Medikaments zur Behandlung von soliden oder liquiden Tumoren.

2. Verwendung nach Anspruch 1, wobei n 0 ist und R₄ COOR₅ ist, wobei R₅ wie in Anspruch 1 definiert ist.

3. Verwendung nach Anspruch 1, wobei n 0 oder 1 ist und R₄ COOH ist.

4. Verwendung nach Anspruch 1, wobei n 1 ist und R₄ COOR₅ ist, wobei R₅ wie in Anspruch 1 definiert ist.

5. Verwendung nach Anspruch 1, wobei n 1 ist und R₄ OH ist.

6. Verwendung nach Anspruch 1, wobei n 1 ist und R₄ 4-Chlorphenylcarbonyloxy ist.

7. Verwendung nach Anspruch 2 oder 4, wobei R₅ C₁-C₆-Alkyl, vorzugsweise Methyl oder Ethyl, ist.

8. Verwendung nach irgendeinem der vorhergehenden Ansprüche, wobei X H ist.

9. Verwendung nach irgendeinem der Ansprüche 1 bis 7, wobei X -CO-R₂ ist, wobei R₂ wie in Anspruch 1 definiert ist.

10. Verwendung nach Anspruch 9, wobei R₂ Phenyl oder Naphthyl ist.

11. Verwendung nach Anspruch 9, wobei R₂ Phenyl ist, das in para-Position mit Cl, F, OCH₃ oder CH₃ monosubstituiert ist.

12. Verwendung nach irgendeinem der Ansprüche 1 bis 11, wobei R₆ und R₇ H sind.

13. Verwendung nach irgendeinem der Ansprüche 2 bis 12, wobei Y SO₂ ist.

14. Verwendung nach Anspruch 13, wobei die Verbindungen aus der Gruppe ausgewählt sind, welche besteht aus:
Ethyl-10,11-dihydropyrrolo[1,2-b][1,2,5]benzothiadiazepin-11-carboxylat-5,5-dioxid (RS 678);
Ethyl-10-(4-tolyl)-10,11-dihydropyrrolo[1,2-b]-[1,2,5]benzothiadiazepin-11-acetat-5,5-dioxid (RS-779);
10,11-Dihydropyrrolo[1,2-b][1,2,5]benzothiadiazepin-11-carbonsäure-5,5-dioxid (RS-735);
10,11-Dihydropyrrolo[1,2-b][1,2,5]benzothiadiazepin-11-essigsäure-5,5-dioxid (RS-791);
10,11-Dihydropyrrolo[1,2-b][1,2,5]benzothiadiazepin-11-methanol-5,5-dioxid (RS-750);
Ethyl-10,11-dihydro-10-(4-tolyl)pyrrolo[1,2-b]-[1,2,5]benzothiadiazepinacetat-5,5-dioxid (RS-769);
Ethyl-10,11-dihydro-10-(benzoyl)pyrrolo[1,2-b]-[1,2,5]benzothiadiazepin-11-carboxylat-5,5-dioxid (RS-2629);
Ethyl-10,11-dihydropyrrolo[1,2-b][1,2,5]benzothiadiazepin-11-acetat-5,5-dioxid (RS-752);
Ethyl-10,11-dihydro-10-(benzoyl)pyrrolo[1,2-b]-[1,2,5]benzothiadiazepin-11-acetat-5,5-dioxid (RS-2645);
10,11-Dihydropyrrolo[1,2-b][1,2,5]-benzothiadiazepin-11-essigsäure-5,5-dioxid (RS-761);
11-(4-Chlorphenylbenzoyloxymethylen)-10,11-dihydropyrrolo[1,2-b][1,2,5]benzothiadiazepin-5,5-dioxid (RS-786);
Ethyl-10,11-dihydro-10-(4-chlorbenzoyl)pyrrolo [1,2-b] [1,2,5]benzothiadiazepin-11-carboxylat-5,5-dioxid (RS-2630);
Ethyl-10,11-dihydro-10-(1-naphthoyl)pyrrolo[1,2-b]-[1,2,5]benzothiadiazepin-11-carboxylat-5,5-dioxid (RS-2631);
Ethyl-10,11-dihydro-10-(4-fluorbenzoyl)pyrrolo[1,2-b]-[1,2,5]benzothiadiazepin-11-carboxylat-5,5-dioxid (RS-2632);
Ethyl-10,11-dihydro-10-(4-chlorbenzoyl)pyrrolo[1,2-b] [1,2,5]benzothiadiazepin-11-acetat-5,5-dioxid (RS-2660);
Ethyl-10,11-dihydro-10-(1-naphthoyl)pyrrolo[1,2-b]-[1,2,5]benzothiadiazepin-11-acetat-5,5-dioxid (RS-2661);
oder
Ethyl-10,11-dihydro-10-(4-fluorbenzoyl)pyrrolo[1,2-b]-[1,2,5]benzothiadiazepin-11-acetat-5,5-dioxid (RS-2652).

15. Verwendung nach irgendeinem der Ansprüche 2 bis 12, wobei Y NR ist und R CH₃ ist.

16. Verwendung nach Anspruch 15, wobei die Verbindungen aus der Gruppe ausgewählt sind, welche besteht aus:
Methyl-10,11-dihydro-5-methyl-5H-pyrrolo[1,2-b]-[1,2,5]benzothiazepin-11-acetat;
Methyl-10,11-dihydro-5-methyl-10-benzoyl-5H-pyrrolo[1,2-b][1,2,5]benzothiazepin-11-acetat (RS-337);
oder Methyl-10,11-dihydro-5-methyl-10-(4-methoxybenzoyl)-5H-pyrrolo[1,2-b][1,2,5]benzothiazepin-11-acetat (RS-421).

17. Verwendung nach irgendeinem der Ansprüche 1 bis 16, wobei die soliden Tumore Karzinome sind, welche aus der Gruppe ausgewählt sind, die aus Eierstock-, Schilddrüsen-, Dickdarm-, Pankreas-, Brust-, Magen-, Prostata-, Lungen-Karzinomen besteht.

18. Verwendung nach irgendeinem der Ansprüche 1 bis 16, wobei die liquiden Tumore aus Leukämien ausgewählt sind.

19. Verwendung nach Anspruch 18, wobei die Leukämie chronische myeloische Leukämie (CML) ist.

20. Verwendung nach Anspruch 18, wobei die Leukämie akute myeloische Leukämie (AML) ist.

21. Verwendung nach Anspruch 18, wobei die Leukämie akute lymphoblastische Leukämie (ALL) ist.

22. Verwendung einer pharmazeutischen Zusammensetzung, die mindestens eine der Verbindungen wie in irgendeinem der Ansprüche 1 bis 16 definiert als aktive(n) Wirkstoff(e) zusammen mit einem oder mehreren pharmalogisch annehmbaren Adjuvantien und/oder Exzipienten umfasst, zur Behandlung solider oder liquider Tumore.

23. Verwendung nach Anspruch 22, wobei die soliden Tumore Karzinome sind, welche aus der Gruppe ausgewählt sind, die aus Eierstock-, Schilddrüsen-, Dickdarm-, Pankreas-, Brust-, Magen-, Lungen-, Prostata-Karzinomen besteht.

24. Verwendung nach Anspruch 22, wobei die liquiden Tumore aus Leukämien ausgewählt sind.

25. Verwendung nach Anspruch 24, wobei die Leukämie chronische myeloische Leukämie (CML) ist.

26. Verwendung nach Anspruch 24, wobei die Leukämie akute myeloische Leukämie (AML) ist.

27. Verwendung nach Anspruch 24, wobei die Leukämie akute lymphoblastische Leukämie (ALL) ist.

28. Derivat-Verbindungen von Benzodiazepinen der Formel (i), wobei die Verbindungen aus der Gruppe ausgewählt sind, welche besteht aus:
Ethyl-10,11-dihydro-10-(benzoyl)pyrrolo[1,2-b]-[1,2,5]benzothiadiazepin-11-carboxylat-5,5-dioxid (RS-2629);
Ethyl-10,11-dihydro-10-(benzoyl)pyrrolo[1,2-b]-[1,2,5]benzothiadiazepin-11-acetat-5,5-dioxid (RS-2645);
Ethyl-10,11-dihydro-10-(4-chlorbenzoyl)pyrrolo[1,2-b]-[1,2,5]benzothiadiazepin-11-carboxylat-5,5-dioxid (RS-2630);
Ethyl-10,11-dihydro-10-(1-naphthoyl)pyrrolo[1,2-b]-[1,2,5]benzothiadiazepin-11-carboxylat-5,5-dioxid (RS-2631);
Ethyl-10,11-dihydro-10-(4-fluorbenzoyl)pyrrolo[1,2-b]-[1,2,5]benzothiadiazepin-11-carboxylat-5,5-dioxid (RS-2632);
Ethyl-10,11-dihydro-10-(4-chlorbenzoyl)pyrrolo[1,2-b]-[1,2,5]benzothiadiazepin-11-acetat-5,5-dioxid (RS-2660);
Ethyl-10,11-dihydro-10-(1-naphthoyl)pyrrolo[1,2-b]-[1,2,5]benzothiadiazepin-11-acetat-5,5-dioxid (RS-2661);
oder
Ethyl-10,11-dihydro-10-(4-fluorbenzoyl)pyrrolo[1,2-b]-[1,2,5]benzothiadiazepin-11-acetat-5,5-dioxid (RS-2652).

## Revendications

1. Utilisation de composés dérivés de benzodiazépines de formule (i): 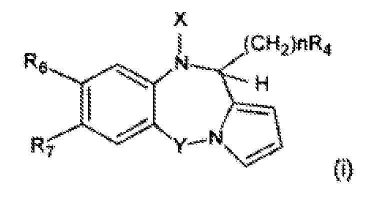 dans laquelle
Y représente SO₂ ou NR, où R représente H ou un groupe alkyle en C₁-C₆;
X représente H, un groupe alkyle en C₁-C₁₂, -CO, -SO, -SO₂ ou -COR₂, SO-R₂, SO₂-R₂, où R₂ est choisi parmi H, un groupe alkyle en C₁-C₁₂, cycloalkyl(en C₃-C₈)alkyle en C₀-C₆, alcényle en C₂-C₆, alcynyle en C₂-C₆, alcoxy(en C₂-C₆)carbonyle, phényle, benzyle, naphtyle, biphényle ou un hétérocycle, chacun, indépendamment des autres, étant substitué en position para, méta ou ortho par 0 à 3 substituants choisis parmi un halogène, -CN, - NH₂, -OH, -NO₂, COOR₃, où R₃ est choisi parmi H, un groupe alkyle en C₁-C₁₂, cycloalkyl(en C₃-C₈)alkyle en C₀-C₆, alcoxy en C₁-C₆, alcényle en C₂-C₆, alcynyle en C₂-C₆, ou alcoxy(en C₂-C₆)carbonyle; un groupe phényle, benzyle, naphtyle, biphényle ou un hétérocycle,
n est égal à 0-6;
R₄ est choisi parmi H, OH, COOH, CN, un groupe alkyle en C₁-C₆, alcoxy en C₁-C₆, alcényle en C₂-C₆, alcynyle en C₂-C₆, alcoxy(en C₂-C₆)carbonyle, -CO, -SO, -SO₂ ou -CO-R₅, SO-R₅, SO₂-R₂, -OCO-R₅, OSO-R₅, OSO₂-R₅, COOR₅, SOOR₅, SO₂OR₅, NMR₅, NHCOR₅, NHSO₂R₅, SR₅, SCOR₅, où R₅ est choisi parmi H, un groupe alkyle en C₁-C₆, alcoxy en C₁-C₆, alcényle en C₂-C₆, alcynyle en C₂-C₆ ou alcoxy(en C₂-C₆)carbonyle, phényle, benzyle, naphtyle, biphényle, ou un hétérocycle, chacun, indépendamment des autres, étant substitué en position para, méta ou ortho par 0 à 3 substituants choisis parmi un halogène, -CN, -NH₂, -OH, -NO₂, COOR₅, où R₅ est choisi parmi H, un groupe alkyle en C₁-C₁₂, cycloalkyl(en C₃-C₈)alkyle en C₀-C₆, phényle, benzyle, naphtyle, biphényle, ou un hétérocycle, un groupe alkyle en C₁-C₆, alcoxy en C₁-C₆, alcényle en C₂-C₆, alcynyle en C₂-C₆, ou alcoxy(en C₂-C₆)carbonyle;
R₆ et R₇ sont choisis indépendamment les uns des autres dans le groupe constitué par H, OH, un halogène, CN, NH₂, NO₂, COOR₃, un groupe alkyle en C₁-C₆, alcoxy en C₁-C₆, alcényle en C₂-C₆, alcynyle en C₂-C₆, et alcoxy(en C₂-C₆)carbonyle;
pour la préparation d'un médicament destiné au traitement de tumeurs solides ou liquides.

2. Utilisation selon la revendication 1, où n est égal à 0 et R₄ représente COOR₅, où R₅ est tel que défini dans la revendication 1.

3. Utilisation selon la revendication 1, où n est égal à 0 ou 1 et R₄ représente COOH.

4. Utilisation selon la revendication 1, où n est égal à 1 et R₄ représente COOR₅, où R₅ est tel que défini dans la revendication 1.

5. Utilisation selon la revendication 1, où n est égal à 1 et R₄ représente OH.

6. Utilisation selon la revendication 1, où n est égal à 1 et R₄ est un groupe 4-chlorophénylcarbonyloxy.

7. Utilisation selon la revendication 2 ou 4, où R₅ est un groupe alkyle en C₁-C₆, de préférence méthyle ou éthyle.

8. Utilisation selon l'une quelconque des revendications précédentes, où X représente H.

9. Utilisation selon l'une quelconque des revendications 1 à 7, où X représente -CO-R₂, R₂ étant tel que défini dans la revendication 1.

10. Utilisation selon la revendication 9, où R₂ représente un groupe phényle ou naphtyle.

11. Utilisation selon la revendication 9, où R₂ représente un groupe phényle monosubstitué en position para par Cl, F, OCH₃ ou CH₃.

12. Utilisation selon l'une quelconque des revendications 1 à 11, où R₆ et R₇ représentent H.

13. Utilisation selon l'une quelconque des revendications 2 à 12, où Y représente SO₂.

14. Utilisation selon la revendication 13, lesdits composés étant choisis dans le groupe constitué par:
le 5,5-dioxyde d'éthyl-10,11-dihydropyrrolo[1,2-b][1,2,5]benzothiadiazépine-11-carboxylate (RS 678);
le 5,5-dioxyde d'éthyl-10-(4-tolyl)-10,11-dihydropyrrolo[1,2-b][1,2,5]benzothiadiazépine-11-acétate (RS-779);
l'acide 10,11-dihydropyrrolo[1,2-b][1,2,5]benzothiadiazépine-11-carboxylique-5,5-dioxyde (RS-735);
l'acide 10,11-dihydropyrrolo[1,2-b][1,2,5]benzothiadiazépine-11-acétique-5,5-dioxyde (RS-791);
le 5,5-dioxyde de 10,11-dihydropyrrolo[1,2-b][1,2,5]benzothiadiazépine-11-méthanol-5,5-dioxyde (RS-750);
le 5,5-dioxyde d'éthyl-10,11-dihydro-10-(4-tolyl)pyrrolo[1,2-b][1,2,5]benzothiadiazépine-acétate (RS-769);
le 5,5-dioxyde d'éthyl-10,11-dihydro-10-(benzoyl)pyrrolo[1,2-b][1,2,5]benzothiadiazépine-11-carboxylate (rus-2629);
le 5,5-dioxyde d'éthyl-10,11-dihydropyrrolo[1,2-b][1,2,5]benzothiadiazépine-11-acétate (RS-752);
le 5,5-dioxyde d'éthyl-10,11-dihydro-10-(benzoyl)pyrrolo[1,2-b][1,2,5]benzothiadiazépine-11-acétate (RS-2645);
l'acide 10,11-dihydropyrrolo[1,2-b][1,2,5]benzothiadiazépine-11-acétique-5,5-dioxyde (RS-761);
le 5,5-dioxyde de 11-(4-chlorophénylbenzoyloxyméthylène)-10,11-dihydropyrrolo[1,2-b][1,2,5]benzothiadiazépine (RS-786);
le 5,5-dioxyde d'éthyl-10,11-dihydro-10-(4-chlorobenzoyl)pyrrolo[1,2-b][1,2,5]benzothiadiazépine-11-carboxylate (RS-2630);
le 5,5-dioxyde d'éthyl-10,11-dihydro-10-(1-naphtoyl)pyrrolo[1,2-b][1,2,5]benzothiadiazépine-11-carboxylate (RS-2631);
le 5,5-dioxyde d'éthyl-10,11-dihydro-10-(4-fluorobenzoyl)pyrrolo[1,2-b][1,2,5]benzothiadiazépine-11-carboxylate (RS-2632);
le 5,5-dioxyde d'éthyl-10,11-dihydro-10-(4-chlorobenzoyl)pyrrolo[1,2-b][1,2,5]benzothiadiazépine-11-acétate (RS-2660);
le 5,5-dioxyde d'éthyl-10,11-dihydro-10-(1-naphtoyl)pyrrolo[1,2-b][1,2,5]benzothiadiazépine-11-acétate (RS-2661);
ou
le 5,5-dioxyde d'éthyl-10,11-dihydro-10-(4-fluorobenzoyl)pyrrolo[1,2-b][1,2,5]benzothiadiazépine-11-acétate (RS-2652).

15. Utilisation selon l'une quelconque des revendications 2 à 12, où Y représente NR et R représente CH₃.

16. Utilisation selon la revendication 15, lesdits composés étant choisis dans le groupe constitué par:
le 10,11-dihydro-5-méthyl-5H-pyrrolo[1,2-b][1,2,5]benzothiazépine-11-acétaté de méthyle;
le 10,11-dihydro-5-méthyl-10-benzoyl-5H-pyrrolo[1,2-b][1,2,5]benzothiazépine-11-acétaté de méthyle (RS-337);
ou le 10,11-dihydro-5-méthyl-10-(4-méthoxybenzoyl)-5H-pyrrolo[1,2-b][1,2,5]benzothiazépine-11-acétate de méthyle (RS-421).

17. Utilisation selon l'une quelconque des revendications 1-16, où lesdites tumeurs solides sont des cancers choisis dans le groupe constitué par les cancers des ovaires, de la thyroïde, du côlon, du pancréas, du sein, de l'estomac, de la prostate, du poumon.

18. Utilisation selon l'une quelconque des revendications 1-16, où lesdites tumeurs liquides sont choisies parmi les leucémies.

19. Utilisation selon la revendication 18, ladite leucémie étant la leucémie myéloïde chronique (LMC).

20. Utilisation selon la revendication 18, ladite leucémie étant la leucémie aiguë myéloblastique (LAM).

21. Utilisation selon la revendication 18, ladite leucémie étant la leucémie aiguë lymphoblastique (LAL).

22. Utilisation d'une composition pharmaceutique comprenant au moins l'un des composés tels que définis dans l'une quelconque des revendications 1 à 16, comme principes actifs, conjointement avec un ou plusieurs adjuvants et/ou excipients acceptables sur le plan pharmacologique, pour le traitement des tumeurs solides ou liquides.

23. Utilisation selon la revendication 22, où lesdites tumeurs solides sont les cancers choisis dans le groupe constitué par les cancers des ovaires, de la thyroïde, du côlon, du pancréas, du sein, de l'estomac, du poumon, de la prostate.

24. Utilisation selon la revendication 22, où lesdites tumeurs liquides sont choisies parmi les leucémies.

25. Utilisation selon la revendication 24, ladite leucémie étant la leucémie myéloïde chronique (LMC).

26. Utilisation selon la revendication 24, ladite leucémie étant la leucémie aiguë myéloblastique (LAM).

27. Utilisation selon la revendication 24, ladite leucémie étant la leucémie aiguë lymphoblastique (LAL).

28. Composés dérivés de benzodiazépines de formule (i), lesdits composés étant choisis dans le groupe constitué par:
le 5,5-dioxyde d'éthyl-10,11-dihydro-10-(benzoyl)pyrrolo[1,2-b][1,2,5]benzothiadiazépine-11-carboxylate (RS-2629);
le 5,5-dioxyde d'éthyl-10,11-dihydro-10-(benzoyl)pyrrolo[1,2-b] [1,2,5]benzothiadiazépine-11-acétate (RS-2645);
le 5,5-dioxyde d'éthyl-10,11-dihydro-10-(4-chlorobenzoyl)pyrrolo[1,2-b][1,2,5]benzothiadiazépine-11-carboxylate (RS-2630);
le 5,5-dioxyde d'éthyl-10,11-dihydro-10-(1-naphtoyl)pyrrolo[1,2-b][1,2,5]benzothiadiazépine-11-carboxylate (RS-2631);
le 5,5-dioxyde d'éthyl-10,11-dihydro-10-(4-fluorobenzoyl)pyrrolo[1,2-b][1,2,5]benzothiadiazépine-11-carboxylate (RS-2632);
le 5,5-dioxyde d'éthyl-10,11-dihydro-10-(4-chlorobenzoyl)pyrrolo[1,2-b][1,2,5]benzothiadiazépine-11-acétate (RS-2660);
le 5,5-dioxyde d'éthyl-10,11-dihydro-10-(1-naphtoyl)pyrrolo[1,2-b][1,2,5]benzothiadiazépine-11-acétate (RS-2661);
ou
le 5,5-dioxyde d'éthyl-10,11-dihydro-10-(4-fluorobenzoyl)pyrrolo[1,2-b][1,2,5]benzothiadiazépine-11-acétate (RS-2652).
